(19) Europäisches Patentamt
European Patent Office
Office européen des brevets

(11) EP 1 144 025 B1

(12) **EUROPEAN PATENT SPECIFICATION**

(45) Date of publication and mention of the grant of the patent:
**25.06.2008 Bulletin 2008/26**

(21) Application number: **00983711.3**

(22) Date of filing: **16.11.2000**

(51) Int Cl.:
***A61M 1/36*** *(2006.01)* ***A61M 1/02*** *(2006.01)*

(86) International application number:
**PCT/US2000/031501**

(87) International publication number:
**WO 2001/036022 (25.05.2001 Gazette 2001/21)**

(54) **METHOD FOR LEUKOREDUCTION OF RED BLOOD CELLS**

VERFAHREN ZUR LEUKOZYTENREDUZIERUNG UNTER ROTEN BLUTKÖRPERCHEN

PROCEDE DE LEUCOREDUCTION DE GLOBULES ROUGES

(84) Designated Contracting States:
**AT BE CH CY DE DK ES FI FR GB GR IE IT LI LU MC NL PT SE TR**

(30) Priority: **17.11.1999 US 166104 P**

(43) Date of publication of application:
**17.10.2001 Bulletin 2001/42**

(73) Proprietor: **Gambro BCT, Inc.**
**Lakewood, CO 80215 (US)**

(72) Inventors:
- **VAN WAEG, Geert**
  **B-1200 Brussels (BE)**
- **GIBBS, Bruce, W.**
  **Arvada, CO 80005 (US)**
- **ANTOON, Marc**
  **B-3000 Leuven (BE)**
- **MCBURNEY, Laura**
  **Lakewood, CO 80215 (US)**

(74) Representative: **Roberts, Mark Peter et al**
**J.A. Kemp & Co.**
**14 South Square**
**Gray's Inn**
**London WC1R 5JJ (GB)**

(56) References cited:

| | |
|---|---|
| **EP-A- 0 243 744** | **EP-A- 0 331 174** |
| **WO-A-01/24848** | **WO-A-99/11305** |
| **DE-A- 4 022 700** | **DE-A- 4 308 880** |
| **FR-A- 2 695 037** | **US-A- 5 954 971** |

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 1 144 025 B1

## Description

The present invention relates to methods for the leukoreduction of red blood cells preferably collected with an apheresis system.

One well-known type of extracorporeal blood processing involves an apheresis system and/or procedure in which blood is removed from a donor, directed to a blood component separation device (*e.g.*, centrifuge), and separated into various blood component types (*e.g.*, red blood cells, white blood cells, platelets, plasma) for collection purposes. One or more or all of these blood component types may be collected before storage, while the remainder may simply be returned to the donor.

A number of factors may affect the commercial viability of an apheresis system. One factor relates to the time and/or expertise required of an individual to prepare and operate the apheresis system. For instance, reducing the time required by the operator to complete an entire collection procedure, as well as reducing the complexity of these actions, can increase productivity and/or lower the potential for operator error. Moreover, reducing the dependency of the system on the operator may further lead to reductions in the credentials desired/required for the operators of these systems.

Performance-related factors also affect the commercial viability of an apheresis system. Performance may be judged in terms of the collection efficiency of the apheresis system, which may impact or improve product quality and/or may in turn reduce the amount of processing time and thus decrease operator burden and increase donor convenience. The collection efficiency of a system may of course be gauged in a variety of ways, such as by the amount of a particular blood component type which is collected in relation to the quantity of this blood component type which passes through the apheresis system. Performance may also be evaluated based upon the effect which the apheresis procedure has on the various blood component types. For instance, it is desirable to minimize the adverse effects on the blood component types as a result of the apheresis procedure (*e.g.*, reduce platelet activation).

Another performance-related factor is the end quality of the collected blood component. For example, if red blood cells are the component to be collected, it is generally desirable that such red blood cells be leukoreduced by the removal of white blood cells or leukocytes. White blood cells can present problems to the ultimate recipient of the collected blood component. Transfused products containing white blood cells can provoke immunogenic reactions and viral diseases. Conventionally, filters have been used to remove leukocytes from collected blood products or components. For example, U.S. Patent No. 5,954,971 discloses the use of a filter with an apheresis system for filtering a diluted blood component prior to collection. Other distinctive methods have also been used, and these have generally dictated special preliminary steps such as pre-chilling and/or overnight storage of collected components prior to filtration. Another distinct conventional filtration step is the venting or air handling/re-circulation or bypassing at the end of the filtration procedure which had been deemed important for substantial recovery of a remainder portion of the blood component to be processed through a red blood cell filter. An apparatus and method for red blood cell filtration in conjunction with apheresis separation is also disclosed in the commonly-owned U.S. Patent Application Serial Number 09/672,519, filed September 27, 2000. Further background on apheresis red blood cell separation and collection can be found in the PCT publication WO99/11305 WO 01/24848, citable under Article 54(3) EPC discloses adding storage solution to red blood cells and filtering with a leukoreduction filter.

The invention is defined in the claims.

An apheresis system which may be used to provide the red blood cells upon which the method of the present invention is carried out, generally includes at least a blood component separation device (*e.g.*, a membrane-based separation device, and/or a rotatable centrifuge element, such as a rotor and channel combination), which provides the mechanism and/or the forces required to separate blood into its various blood component types (*e.g.*, red blood cells, white blood cells, platelets, and/or plasma). The separation device can include a centrifuge channel which receives a disposable blood processing vessel. Typically, before carrying out the claimed method, a donor is fluidly interconnected with the blood processing vessel by an extracorporeal tubing circuit, and preferably the blood processing vessel and extracorporeal tubing circuit collectively define a closed, sterile system. When the fluid interconnection is established, blood may be extracted from the donor and directed to the blood component separation device such that at least one type of blood component may be separated and removed from the blood for collection.

The red blood cells may be provided by separating high hematocrit red blood cells from blood within a blood processing vessel of a blood component separation machine and collecting at least a portion of the separated red blood cells within a red blood cell collection container that is disparate from yet preconnected via tubing lines to the blood processing vessel. Such red blood cells may be separated and collected alone, or prior or subsequent to or concurrently with other blood components such as platelets and/or plasma. According to the present invention, before the ultimate collection of the red blood cells in the collection container, the red blood cells are filtered through a filtration device.

The invention includes rinsing or flushing a storage solution through the leukoreduction filter after completion of the red blood cell filtration through the leukoreduction filter.

According to the invention, the separated red blood cells are filtered in a high hematocrit state as they exist after separation in the apheresis system. A storage solution is added to the red blood cells after such filtration. The storage solution is also flushed through the filter after the red blood cells are filtered therethrough.

These and still further aspects of the present invention are more particularly described in the following description of the preferred embodiments presented in conjunction with the attached drawings which are described briefly below.

Fig. 1 is a schematic view of one embodiment of an apheresis system which can be used to obtain red blood cells for processing by the method of the present invention;

Figs. 2A-2B illustrate an extracorporeal tubing circuit, cassette assembly, and filter and collection bag assembly thereof for use with the system of Fig. 1 pursuant to the present invention;

Fig. 2C illustrates an alternative extracorporeal tubing circuit filter and collection bag assembly for use according to the present invention;

Fig. 3 is another schematic view of an apheresis system together with the filter and collection bag assembly as depicted in Figs. 2A and 2B;

Fig. 4A is a first side elevational view of an apheresis system such as that shown in Fig. 1 used with a filter and collection bag assembly;

Fig. 4B is a second side elevational view of an apheresis system and a filter and collection bag assembly as in Fig. 4A with an alternative placement of containers and tubing lines; and

Figs. 5A-5G are representations of data gathered as described relative to Examples A to E.

The present invention will be described in relation to the accompanying drawings which assist in illustrating the pertinent features hereof.

A preferred blood apheresis system **2** for providing red blood cells for processing by the present invention is schematically illustrated in Fig. 1. System **2** preferably provides for a continuous blood component separation process. Generally, whole blood is withdrawn from a donor **4** and is substantially continuously provided to a blood component separation device **6** where the blood is continuously separated into various component types and at least one of these blood component types is preferably continuously collected from the device **6.** These blood components may then be provided for collection and subsequent use by another through transfusion or may either be uncollected and then be returned to the donor **4.**

In the blood apheresis system **2,** blood is withdrawn from the donor **4** and directed through a preconnected disposable set **8** which includes an extracorporeal tubing circuit **10** and, in the preferred embodiment, a blood processing vessel **352** which together define a completely closed, sterile and disposable system. The disposable set **8** is mounted on and/or in the blood component separation device **6** which preferably includes a pump/valve/sensor assembly 1000 for interfacing with the extracorporeal tubing circuit **10**, and a channel assembly **200** for interfacing with the disposable blood processing vessel **352**.

The channel assembly **200** may include a channel housing **204** which is rotatably interconnected with a rotatable centrifuge rotor assembly **568** which provides the centrifugal forces required to separate blood into its various blood component types by centrifugation. The blood processing vessel **352** may then be interfitted within the channel housing **204**. When thus connected as described, blood can then be flowed substantially continuously from the donor **4**, through the extracorporeal tubing circuit **10**, and into the rotating blood processing vessel **352**. The blood within the blood processing vessel **352** may then be continuously separated into various blood component types and at least one of these blood component types (*e.g.*, platelets, plasma, or red blood cells) is preferably continually removed from the blood processing vessel **352**. Blood components which are not being retained for collection (*e.g.*, platelets and/or plasma) are preferably also removed from the blood processing vessel **352** and returned to the donor **4** via the extracorporeal tubing circuit **10**. Note, various alternative apheresis systems (not shown) may also be used; including batch processing systems (non-continuous inflow of whole blood or outflow of separated blood components) or smaller scale RBC/plasma separation systems, even if no blood components may be returned to the donor.

Operation of the blood component separation device **6** is preferably controlled by one or more processors included therein, and may advantageously comprise a plurality of embedded computer processors to accommodate interface with ever-increasing PC user facilities (*e.g.*, CD ROM, modem, audio, networking and other capabilities). Relatedly, in order to assist the operator of the apheresis system **2** with various aspects of its operation, the blood component separation device **6** preferably includes a graphical interface **660** preferably with an interactive touch screen **664**.

Further details concerning the operation of a preferred apheresis system, such as the COBE Trima® System (available from the assignee of this application, Gambro, Inc., Lakewood Colorado) may be found in a plurality of publications, including, for example, WO99/11305 and U.S. Patents No. 5,653,887; No. 5,676,644; No. 5,702,357; No. 5,720,716; No. 5,722,946; No. 5,738,644; No. 5,750,025; No. 5,795,317; No. 5,837,150; No. 5,919,154; No. 5,921,950; No. 5,941,842; and No. 6,129,656; among numerous others. A plurality of other known apheresis systems may also be useful herewith, as for example, the Baxter CS3000® and/or Amicus® and/or Autopheresis-C® systems, and/or the Haemonetics MCS® or MCS®+ and/or the Fresenius COM.TEC™ or AS-104™ and/or Dideco or like systems.

<u>Disposable Set: Extracorporeal Tubing Circuit</u>

As illustrated in Figs. 2A-2B, a preferred preconnected extracorporeal tubing circuit **10** may include a cassette assembly **110** and a number of tubing/collection assemblies **20**, **50**, **60**, **80**, **90**, **950** and **100** interconnected therewith. Preferably, a blood removal/return tubing assembly **20** provides a single needle interface between a donor **4** and the remainder of the tubing circuit **10** (although a two-needle set-up may also be used). The preferred embodiment includes a cassette assembly **110**, which is interconnected between the tubing assembly **20** which connects the donor **4** thereto, and blood inlet/blood component tubing line sub-assembly **60** which provides the interface between cassette assembly **110** and blood processing vessel **352**. An anticoagulant tubing assembly **50**, a platelet collection tubing assembly **80**, a plasma collection tubing assembly **90**, a red blood cell collection assembly **950** and a vent bag tubing line sub-assembly **100** are also preferably interconnected with cassette assembly **110** in this embodiment. As will be appreciated, the extracorporeal tubing circuit **10** and blood processing vessel **352** are preferably pre-interconnected to combinatively yield a closed, pre-sterilized disposable assembly for a single use.

The disclosures of the above-listed patents include numerous further details of the preferred apheresis system for use with the present invention. Such details are not repeated here except for certain of those which may relate particularly to red blood cell (hereafter, RBC) collection and/or other RBC processes.

For example, emanating from vessel **352** is an RBC outlet tubing line **64** of the blood inlet/blood component tubing assembly **60** which is interconnected with integral RBC passageway **170** of cassette **115** of cassette assembly **110** (see Fig. 2A). The integral RBC passageway **170** includes first and second spurs **170a** and **170b**, respectively. The first spur **170a** is interconnected with RBC return tubing loop **172** to return separated RBCs to a donor **4.** For such purpose, the RBC return tubing loop **172** is preferably interconnected to the top of a blood return reservoir **150** of the cassette assembly **110**. The second spur **170b** may, as preferred herein, be connected with an RBC collection tubing assembly **950** (see Figs. 2A and 2B) for collecting RBCs during use. RBC collection tubing assembly **950** preferably includes RBC collector tubing line **952** which communicates with spur **170b**, an intermediate RBC collection reservoir or bag **954**, an RBC filtration sub-assembly including an ultimate RBC collection reservoir or bag **958**, an RBC leukoreduction filter **960** and an air removal bag **962**. A sterile barrier filter/drip spike assembly **956** preferably including a sterile barrier **957** and a spike **959**, may also be included for connecting to a source of storage solution, *inter alia,* and may be connected to RBC bag **954** through tubing line **955** and an optional frangible connector **968** as will be described in more detail below. Bags **954** and **958** are connected to each other by two tubing lines **964**, **965** between and to each of which the RBC leukoreduction filter **960** is connected. A clamp **966** may be included on line **965**. Collection bag **954** may be, in one less preferred embodiment, interconnected to RBC filter **960** through a frangible connector **967**. The air removal bag **962** is attached to the RBC collection bag **958** by a tubing line **961** which may have a clamp **963** attached thereto. The RBC collection tubing line, filter and container sub-assembly **950** is preferably a preconnected part of the disposable assembly 8/10.

An alternative tubing set filter and collection bag assembly **950a** is shown in Fig. 2C and includes a second collection bag **958a** connected via a Y-type of connection to filter **960,** via the branch tubing line **965a**. A further air bag **962a** is preferably connected to the second bag 958a via a tubing line **961a**. More details particularly as to the use hereof will be set forth below.

Most portions of the tubing assemblies **20**, **50**, **60**, **80**, **90**, **100** and **950** and cassette assembly **110** are preferably made from plastic components including, for example, polyvinyl chloride (PVC) tubing lines, that permit visual observation and monitoring of blood/blood components therewithin during use. It should be noted that thin-walled PVC tubing may be employed for approved, sterile docking (i.e., the direct connection of two pieces of tubing line) for the RBC collector tubing lines **952**, **964** and **965** (as necessary or desired and/or for an RBC storage solution spike assembly b), *inter alia.* All tubing lines are preconnected before sterilization of the total disposable assembly to assure that maximum sterility of the system is maintained. Note, a highly desirable advantage to preconnection of all of the elements of the tubing circuit including the filter and collection bag sub-assembly **950** involves the complete pre-assembly and then sterilization hereof after assembly such that no sterile docking is later necessary (spike addition of storage solution excepted). Thus, the costs and risks of sterile docking are eliminated. Alternatively, thicker-walled PVC tubing may be employed for approved, sterile docking RBC collector tubing lines **952**, **964** and **965**, *inter alia.*

As mentioned, a cassette assembly **110** may be mounted upon and operatively interface with the pump/valve/sensor assembly **1000** of blood component separation device **6** during use. Further details of an apheresis system set-up including the loading and interaction of a disposable assembly **8**/**10** with a blood component separation device **6**, may be found in the above-listed patents, *inter alia*, and are not exhaustively repeated here.

Operation of Extracorporeal Tubing Circuit and Blood Component Separation Device

Priming and various other operations of the apheresis process are preferably carried out as set forth in the above-listed patents, *inter alia*. However, certain basic features are also described generally here with particular reference to the schematic diagram of Fig. 3, as well as with continuing reference to Figs. 1, 2A and 2B.

For example, during a blood removal submode, whole blood will be passed from a donor **4** into blood removal/return tubing assembly b and is then transferred to blood component separation device **6** (see generally Fig. 3). At device **6**, the blood is flowed to the processing vessel **352** (schematically shown in dashed lines in Fig. 3) via the cassette assembly **110** and line **62** of the blood inlet/blood component tubing assembly **60** (Figs. 1 and 2A). Separation processing then occurs preferably on a substantially continuous basis in vessel **352**; i.e., blood continuously flows therein, is continuously separated and continuously flows as separated components therefrom. After separation processing in vessel **352** (though separation is continuously occurring), uncollected blood components are transferred from the processing vessel **352** to and through cassette assembly **110**, into and may then accumulate in reservoir **150** (Fig. 2A) of cassette **110** up to a predetermined level at which the blood component separation device **6**, in a single needle operation, may (though in a continuous system, need not) pause the blood removal submode and initiate a blood return submode wherein these uncollected and/or treated components are returned to the donor **4**. As such, these accumulated components may be transferred into the blood return tubing line of blood removal/return tubing assembly **20** and back into the donor **4**. During the single needle blood return mode, when the accumulated return blood components in reservoir **150** are removed down to a predetermined level, blood component separation device **6** will then automatically end the blood return submode. This preferably will also automatically serve to reinitiate the blood removal submode. The cycle between blood removal and blood return submodes will then continue until a predetermined amount of RBCs or other collected blood components have been harvested. In an alternative dual needle scheme, as is known in the art, blood may be continually removed from and blood components continually returned to a donor **4**. Note, the detailed mechanisms for such operations, including controlling the pumps, for example, are not shown or described in detail herein, particularly not in the schematic view of Fig. 3.

With specific reference to Fig. 2A, in normal operation, whole blood will pass from the donor **4** through the needle and blood removal tubing assembly **20**, cassette assembly **110** and blood inlet tubing line **62** to processing vessel **352**. The whole blood will then be separated in vessel **352**. A platelet stream may be separated herein and be either collected in collector assembly **80** or diverted to reservoir **150**. Similarly, separated plasma may also be separated in vessel **352** and either be collected in the container of plasma tubing assembly **90** or diverted to reservoir **150**. Further, red blood cells (including potentially some white blood cells) may be separated in and passed from vessel **352** through RBC outlet tubing line **64**, through cassette assembly **110** and, in return mode, into reservoir **150**. In the preferred alternative, during an RBC collection procedure described hereinbelow, separated RBCs will be delivered to RBC collector tubing assembly **950** through tubing line **952** for collection.

Further details of apheresis processing for the separation of blood into its components may be found in the above-listed patents *inter alia* and are not substantially repeated here. It may be noted, however, that although alternative separation mechanisms exits, centrifugation is the preferred separation process which is preferably effected by a channel assembly **200** rotated, for example, by a centrifuge rotor assembly **568** in a device **6** (see Fig. 1). Channel assembly **200** would then preferably include a channel housing **204** which would receive the disposable blood processing vessel **352** of tubing circuit **10** (see Figs. 1 and 2A).

Apheresis Protocol

One preferred protocol, which may be followed for performing an apheresis procedure relative to a donor **4** utilizing the described system **2**, will now be summarized. Initially, an operator loads the disposable plastic assembly **8** in and/or onto the blood component separation device **6**. According hereto, the operator hangs the various bags (*e.g.*, collection bags **954** and **958**, see Fig. 4A, described further below) on the respective hooks (see hook **980** of Fig. 4A, *e.g.*) of the blood component separation device **6**. If one is used, the operator then also loads the cassette assembly **110** on the machine **6** and/or the blood processing vessel **352** within the channel housing **204** as mounted on the centrifuge rotor assembly **568**.

With the extracorporeal tubing circuit **10** and the blood processing vessel **352** loaded in the described manner, the donor **4** may then be fluidly interconnected with the extracorporeal tubing circuit **10** by inserting an access needle of the needle/tubing assembly **20** into the donor **4** (see, *e.g.,* Fig. 3). In addition, the anticoagulant tubing assembly **50** is primed (not shown), and blood removal/return tubing assembly **20** is primed preferably with blood from the donor **4** as described in the above-listed patents, *inter alia.* The blood processing vessel **352** is also primed for the apheresis procedure, preferably also according to the processes described in the same patents. In one example, a blood prime may be used in that blood will be the first liquid introduced into the blood processing vessel **352**. During the priming procedure, as well as throughout the remainder of the apheresis procedure, blood is continuously flowed into the vessel **352**, blood component types are preferably continuously being separated from each other and are also continuously removed from the blood processing vessel **352**, on a blood component type basis. Preferably, at all times during the apheresis procedure, from priming onward, a flow of blood is substantially continuously provided to the blood processing vessel **352** and at least one type of separated component is continually removed.

As RBCs are the component of the most interest in the current invention, the separation protocol will continue with a description of the collection hereof.

The preferred blood apheresis system **2** provides for contemporaneous separation of a plurality of blood components during blood processing, including at least the separation of red blood cells (RBCs) and plasma, but may also provide for the separation and collection of platelets (as shown here), *inter alia.* In turn, such separated blood components may be selectively collected in corresponding storage reservoirs or immediately or after a minor delay returned to the donor **4** during respective blood return submodes (or constantly in a two-needle setup). In this regard, and in one approach where more than one blood component is to be collected, such as both plasma (and/or platelets) and RBCs, blood apheresis system **2** may be used to collect plasma (and if desired separated platelets), during a time period(s) separate from the collection of red blood cells. These components may also be collected simultaneously.

In any event, the RBC collection procedure is preferably controlled via control signals provided by blood collection device **6**. Such an RBC collection procedure may include a setup phase and a collection phase. During such a setup phase, the blood apheresis system **2** may (as in the preferred embodiment) be adjusted automatically to establish a predetermined hematocrit in those portions of the blood processing vessel **352** and extracorporeal tubing circuit 10 through which separated RBCs will pass for collection during the RBC collection phase. A desirable resulting hematocrit for RBC collection may be between 70 and about 90 or even to 95+, and may preferably be established at about 80. Additionally, blood component device **6** may, during the set-up phase, divert the flow of separated RBCs flowing through outlet tubing line **64** through return tubing loop **172** and into blood return reservoir **150** until the desired hematocrit is established. Then, blood component separation device **6** may also selectively control the diversion of the platelets and plasma into reservoir **150** for return to the donor **4**.

In order to establish the desired packing factor and/or hematocrit for the separated RBCs, the operating speed of centrifuge rotor assembly **568** may be selectively established via control signals from blood component separation device **6**, and the blood inlet flow rate to vessel **352** may be selectively controlled by blood component separation device **6** controlling the speeds of the respective pump assemblies (not shown or described in detail here). More particularly, increasing the rpms of centrifuge rotor assembly **568** and/or decreasing the inlet flow rate will tend to increase the packing factor and/or hematocrit, while decreasing the rpms and/or increasing the flow rate will tend to decrease the packing factor and/or hematocrit. As can be appreciated, the blood inlet flow rate to vessel **352** may effectively be limited by the desired packing factor or hematocrit.

To establish a desired anticoagulant (AC) ratio, blood component separation device **6** provides appropriate control signals to the anticoagulant pump so as to introduce anticoagulant into the blood inlet flow at a predetermined rate. Relatedly, it should be noted that the inlet flow rate of anticoagulated blood to blood processing vessel **352** is limited by a predetermined, maximum acceptable anticoagulant infusion rate (ACIR) to the donor **4**. As will be appreciated by those skilled in the art, the predetermined ACIR may be established on a donor-specific basis (*e.g.* to account for the particular total blood volume of the donor **4**). To establish the desired total uncollected plasma flow rate out of blood processing vessel **352**, blood collection device **6** provides appropriate control signals to the plasma (and platelet) pump assembly(ies). This may also serve to increase the hematocrit in the separated RBCs.

In one preferred example, the desired high hematocrit for the separated RBCs will be between about 75 and about 85 and will preferably be about 80; although, again higher hematocrits may be available as well. Then, where a preferred centrifuge rotor assembly **568** defines a rotor diameter of about 10 inches, and where a blood processing vessel **352** is utilized, as described hereinabove, it has been determined that in one preferred embodiment channel housing **204** can be typically driven at a rotational velocity of about 3000 rpms to achieve the desired RBC hematocrit during the setup and red blood cell collection phases. Correspondingly, the blood inlet flow rate to vessel **352** may preferably be established at below about 64.7 ml/min. The desired hematocrit can be reliably stabilized by passing about two whole blood volumes of vessel **352** through vessel **352** before the RBC collection phase is initiated.

To initiate the RBC collection phase, blood component separation device **6** provides an appropriate control signal to the RBC divert valve assembly so as to direct the continuous outflow of the separated high hematocrit RBCs removed from blood processing vessel **352** into the intermediate RBC reservoir **954** through tubing line **952**.

As may be appreciated, in the preferred embodiment, the separated RBCs are preferably not pumped out of vessel **352** for collection, but instead are flowed out vessel **352** and through extracorporeal tubing circuit **10** by the pressure of the blood inlet flow to vessel **352**. Consequently, trauma to the collected RBCs is preferably minimized.

During the RBC collection phase, the inlet flow into vessel **352** is limited by the above-noted maximum acceptable ACIR to the donor **4**. The desired inlet flow rate is also limited by that necessary to maintain the desired packing factor and/or hematocrit, as also discussed. In this regard, it will be appreciated that relative to the setup phase, the inlet flow rate may be adjusted slightly upwards during the RBC collection phase since not all anticoagulant is being returned to the donor **4**. That is, a small portion of the AC may remain with the small amount of plasma that is collected with the high hematocrit RBCs in RBC reservoir **954**.

The above describes how high hematocrit RBCs may be obtained so that they may be processed by the method of the present invention.

According to the present invention, the high hematocrit (high-crit) RBCs are filtered after having been separated within vessel **352**.

Note, the phrase freshly-separated is intended to describe the newly-separated blood components in and as they emerge from the mechanical separation system such as device 6 and processing vessel **352**. It also includes the state of these same separated components for a reasonable length of time after removal from the mechanical separation device such as from vessel **352**. Thus, for example, a first reasonable length of time may include the entire apheresis procedure which may last up to (and perhaps exceed) two (2) hours after which filtration may be begun. Another example may involve a situation in which a red blood cell collection center may, for certain reasons, determine to intermediately collect red blood cell products (in containers **954**, e.g.), and then further process/filter these cumulatively in another location (such as the lab) or at set times, as, for example, once or twice each day (thus filtering up to four (4) or perhaps even eight (8) hours after intermediate collection). If reasonable (though not preferred), this time shift could conceivably stretch to even the next day (24 or 36 hours) before the subsequent processing/filtration of the still substantially freshly-separated red blood cell product. Freshly-separated is not intended to refer to previously stored separated red blood cell components. Two further terms used herein have similar distinctions, namely, "recently removed" and "soon after." Recently removed is referred to herein primarily relative to that blood taken from the donor which may be immediately taken and processed in a mechanical separation system, or which may have been taken and held subject to a reasonable non-storage type of delay prior to separation processing. Similarly, "soon after" is used in like manners relative to both of these circumstances as well, as, for example, when separated blood components may be removed from the separation vessel, *e.g.* soon after separation (whether in continuous or batch mode).

In any event, from the standpoint of the donor **4** and machine **6**, following the separation and intermediate collection processes of the desired quantity of red blood cells, blood separation device **6** may then provide a control signal to the RBC divert assembly so as to divert any further RBC flow back to the donor **4**. Additionally, if further blood processing, by apheresis centrifugation here, is not desired, rinseback procedures may be completed. Additionally, once the minimum desired RBCs have been diverted into assembly **950** and before, during or preferably after filtration completion, the intermediate red blood cell reservoir **954** (and thus the entire sub-assembly **950**) may then be disconnected from the extracorporeal tubing circuit **10**. If not already begun or even completed, the filtration process may then begin, as described in more detail below. According to the present invention, a storage solution will, after filtration of the RBCs, then be added to the intermediate red blood cell reservoir or bag **954** through a spike connection to a storage solution bag **970** (see Fig. 3) through spike **959**, and if used, the opening of the optional frangible connector **968** (see Fig. 2B). This process will also be described further below. Such storage solution may advantageously facilitate storage of the RBCs for up to about 42 days at a temperature of about 1-6 degrees C. In this regard, acceptable storage solutions include a storage solution generically referred to in the United States as Additive Solution 3 (AS-3), available from Medsep Corp. located in Covina, California; and/or a storage solution generically referred to in Europe as SAG-M, available from MacoPharma located in Tourcoing, France.

The storage solution may be and preferably is contained in a separate storage solution bag **970** that can be selectively later interconnected to the intermediate RBC bag **954**, preferably through a spike connection **956**. In an alternative embodiment, such selective interconnection may be provided via sterile-docking to tubing line **955** as an example (process not shown) utilizing a sterile connecting device (not shown). By way of example, one such sterile connecting device to interconnect tubing line **955** between the storage solution container **970** and the intermediate bag **954**, is that offered under the trade name "TSCD" or "SCD™ 312" by Terumo Medical Corporation located in Somerset, New Jersey. In the preferred alternative, as introduced above, the selective interconnection may be established utilizing a sterile barrier filter/spike assembly **956**. The use of such a sterile barrier filter/spike assembly **956** facilitates the maintenance of a closed system, thereby effectively avoiding bacterial contamination. By way of example, the mechanical, sterile barrier **957** filter in such an assembly **956** may include a porous membrane having 0.2 micron pores. A frangible connector **968** (Fig. 2B) may be provided as a further option for selectively opening tubing line **955** for introduction of the storage solution into the RBC filter system.

In order to ensure the maintenance of RBC quality, the intermediate and collection RBC bags **954**, **958** the storage solution and the anticoagulant used during blood processing should be compatible. For example, the intermediate and collection RBC reservoirs **954**, **958** may be a standard PVC DEHP reservoir (i.e. polyvinyl chloride-diethylhexylphthallate) such as those offered by the Medsep Corporation. Alternatively, a citrated PVC reservoir may be employed. Such a reservoir may utilize a plasticizer offered under the trade name "CITRIFLE3C-B6" by Moreflex located in Commerce, California. Further, the anticoagulant utilized in connection with the above-described red blood cell collection procedures may be an acid citrate dextrose-formula A (ACD-A).

Nevertheless, according to the present invention as introduced above, before the storage solution is to be added to the collected red blood cells, selective filtering will be performed to remove white blood cells therefrom. More particularly leukoreduction filtering is desired to establish a white blood cell count of at least < 5 x $10^6$ white blood cells/unit (*e.g.* about 250 ml.) to reduce any likelihood of febrile non-hemolytic transfusion reactions. Moreover, such filtering will more desirably achieve a white blood cell count of < 1 x $10^6$ white blood cells/unit to reduce any risk of HLA (i.e. human leukocyte A) sensitization and/or other serious side reactions. Studies have also shown positive effects for pre-storage leukocyte reduction in improving the functional quality of erythrocytes during storage and in decreasing the occurrence of alloimmunization in patients receiving multiple transfusions, as well as being favorable in metabolism reactions such as intra-erythrocyte ATP and/or extracellular potassium levels declining more slowly in filtered products. Perhaps more important is the reduction of transfusion transmitted disease, especially cytomegalovirus (CMV) and/or HIV, *inter alia*.

Accordingly, the intermediate red blood cell container **954** is, in the preferred embodiment, pre-connected to a red cell filter/collection bag sub-assembly as is shown in Figs. 1, 2A and 2B (and 2C) so that high hematocrit (preferably Hct approximately equal to or greater than 80), freshly separated red blood cells are preferably gravity transferred from the intermediate bag **954** through filter **960** and into the ultimate RBC collection bag **958**. Gravity drainage filtration is shown in Figs. 3 and 4A, as will be described further below. The red cell filter and collection bag sub-assembly is preferably preconnected to the intermediate bag **954** as part of the disposable assembly **10** (to avoid the costs and risks of sterile docking) as shown in Figs. 1, 2A and 2B in accordance with the teachings of this invention, or may be added to the previously existing disposable systems to form a post-manufacturing-connectable disposable assembly using commercially available filter/bag kits such as those available under the trade names "r\LS" manufactured by HemaSure, Inc. located in Marlborough, Massachusetts, or "Sepacell" from Asahi Corp and/or Baxter, Inc. and/or "RC 100", "RC50" and "BPF4" from Pall Corp. located in Glencove, New York, *inter alia.* In either event, the red cell filter/bag sub-assembly is preferably connected (pre- or post-) to the intermediate bag **954** through a tubing line **964** as shown. In one embodiment, this connection contains a frangible connector **967**; however, in another and herein preferred embodiment, no such flow stopping connector is disposed between the intermediate bag **954** and filter **960** so that filtration may begin as soon as a flow of freshly separated RBCs reach the intermediate bag **954** as described herein. Note, frangible connector **967** or any other sort of flow stopping mechanism such as a valve or a clamp is an option in lieu of an open line to provide the option of preventing flow directly to the filter **960**. This option allows for filtering at some point in time later than simultaneously with or during the intermediate collection in bag **954**. Such delayed filtering could be soon after intermediate collection as defined hereinabove.

Nevertheless, referring now primarily to Figs. 2B, 3 and 4A, the preferred procedure for the filtration of RBCs freshly separated and collected from the apheresis process is as follows. These freshly separated RBCs are still in an undiluted, high-hematocrit state (Hct approximately 80) during the preferred filtration process.

The RBC collection filtration system **950** is activated to filter the RBCs. The RBC sub-assembly **950** can be severed from the extracorporeal tubing circuit 10 at tubing line **952** prior to such filtration (see description below). Otherwise, such severing will be performed later in the process.

In either case; the high-crit RBCs are flowed preferably by gravity drainage through filter **960**. As such, intermediate bag **954** is preferably hung at a level above both the collection bag **958** and the filter **960** (see Figs. 3 and 4A), and frangible connector **967** is then opened (if such a connector or a like optional flow-stopping member is included in sub-assembly **950**) so that the collected or continuously collecting high-crit RBCs are allowed to gravity drain downwardly from bag **954** through the filter **960** and into the collection bag **958**. A preferred embodiment of this is shown in Fig. 4A, where the intermediate bag **954** is hung from a hook **980** of the machine **6** in known fashion. Tubing line **964** depends downwardly therefrom and is shown as connected to the filter **960**, below which depends the next tubing line **965** which is ultimately connected to the collection bag **958** hung from a hook **981** preferably at or near the lowest practical point on the machine **6**. Note, bag **954** is shown still connected via tubing line **952** to highlight the preference of continuing to receive freshly-separated RBCs even though filtration has preferably begun.

Any air from bag **958**, or air caught between the incoming RBCs and bag **958** is ultimately removed to air removal bag **962** through tubing line connection **961**. It is also understood that removal of air may also be achieved by other known (though less desirable here) methods, including, for example, hydrophobic vents and/or by-pass lines. It is desirable to perform the filtering of the RBCs according to the present invention directly on the machine **6** without pre-cooling or pre-storing the RBCs. In such a case, these procedures are thus performed without the previously conventional steps of cooling and storing overnight at 4 degrees Centigrade.

Then, after completion of the filtration, storage solution is then added to the intermediate bag **954** through tubing line **955**. Again, this is preferably done after completion of the filtration of the high hematocrit, non-diluted RBCs through filter **960**. Then, after a storage solution bag **970** has been connected (by spike or sterile welding), as depicted in Figs. 3 and 4B, the frangible connector **968** is opened (if such an optional flow-stopping member is used; see Fig.

2B) to allow the introduction of the storage solution into the emptied bag **954**. The pathway through frangible connector **967** (again, if used) and line **964** remains opened at this point in the procedure and thus the storage solution will flow unabatedly from bag **954** through filter **960** and into the lower collection bag **958** to there mix with and dilute the now filtered high-crit RBCs. Again, all of the steps in operating the RBC filtration system **950** need not be subjected to a cooled, time-delayed environment, such as the 4 degrees Centigrade overnight procedures previously thought necessary.

A preferred embodiment of this subsequent storage solution addition step is shown in Fig. 4B, wherein the intermediate bag **954** is shown removed from the upper hook **980** and re-hung on a mid-level hook **982**. Then, a storage solution bag **970** can be hung from the upper hook **980** so that when connected and hung as shown in Fig. 4B, storage solution can flow down through tubing line **955** and sterile barrier **957** into intermediate bag **954**, from which the storage solution will then flow downwardly from bag **954** through filter **960** and then ultimately into collection bag **958**. Note, the embodiment shown in Fig. 4B also includes a depiction of the severed dis-connection of intermediate bag **954** from inlet tubing line **952**. A stub **952a** remains projecting from bag **954**. This serves to help depict the herein preferred method of storage solution addition only after at least the procedure for blood separation and intermediate collection of high-crit RBCs from vessel **352** is complete. No more separated RBCs are added to the intermediate bag **954** by this point in the procedure; *i.e.,* by the point of adding storage solution. The further preferred steps of having drained all of the RBCs out of bag **954** and having completed filtration thereof through filter **960** prior to the addition of storage solution to bag **954** is not as easily nor separately shown in the Figs. In either event, such a separation may be made by RF sealing the tubing line **952** and then separating in accordance with U.S. Patent Nos. 5,345,070 and 5,520,218, *inter alia*, along the RF-sealed portion of tubing line. Other well known methods can also be used to close the tubing line and then also separate the RBC collection system **950** from the remainder of the disposable assembly **10**. The RBC collection system **950** which would be remaining after such a severing is shown schematically in Fig 2B.

The preferred use of the optional two collection bag assembly **950a** as shown in Fig. 2C is not much different from the above process. Gravity flow down from intermediate bag **954** through filter **960** to and through each of the branch lines **965**, **965a** could be used to fill both collection bags **958**, **958a** simultaneously, or one at a time (wherein a flow stopping member such as a clamp (not shown) could be used to selectively arrest flow into first one then the other of bags **958**, **958a** until full). Then, however, when a desired double product is filtered and collected accordingly, it may be preferred to provide more control over the storage solution flush and addition process. First, it may be desirable to ensure that the two bags **958**, **958a** have substantially equal collected volumes, by weight or other means. Excess from one bag may be manipulated into the other bag, by hand compression for example, to flow through the adjoining tubing lines **965**, **965a**. Then, it may be desired to deliver known amounts of storage solution into the respective bags **958**, **958a**, via clamping first one tubing line **965**, **965a**, and then the other during the flush of storage solution through filter **960**. Removal of air from the two collection bags into respective air bags **962**, **962a** would occur as before. Note, the preferred alternative here involves only a single filter **960** for processing the RBCs for both bags **958**, **958a**. However, a second filter **960a** (shown in dashed lines in Fig. 2C) may alternatively be used herewith as well. As shown, intermediately collected RBCs could be made to flow down from intermediate bag **954** through a first filter **960** into a first collection bag **958**, until this bag is filled. Then, flow down from bag **954** could be diverted to flow through the alternative second filter **960a** to be collected in the second bag **958a**. Other alternatives for double RBC product filtration will also be apparent, as for example having separate first and second intermediate bags **954** (not shown), to which separate filters **960**, **960a** could be attached with their respective collection bags **958**, **958a** *inter alia.*

Several advantages can be realized utilizing the preconnected disposable assembly and the above-described procedure for high-crit red blood cell collection and filtration. Such advantages include: consistency in final RBC product volume and hematocrit; reduced exposure of a recipient if multiple units of blood products are collected from a single donor and transfused to a single recipient; reduced time requirements for RBC collection and filtration, including collection of double units of red blood cells if desired, and reduced risks of bacterial and leukocyte contamination. More particularly, several of the reasons why this high-hematocrit (high-Hct or high-crit) with storage solution (*e.g.*, SAG-M) wash approach would not have appeared to work included the expected slow flow of high hematocrit RBCs through the filter **960**; the expected risk of blocking the filter **960** with the high-crit RBCs; the previously unknown leukodepletion levels at this high hematocrit; and the apparently likely "wash-out" of WBCs by the storage solution (*e.g.*, SAG-M) through filter **960**.

It was conceived and determined to test for possible high-crit filtration success anyway even though the prospect for success appeared unlikely at the outset. The results put serious doubts on the above negative expectations as it was found that: the high-crit RBC units filtered between 10 and 40 minutes, very often between 12 and 18 minutes; leukodepletion before storage solution (SAG-M) wash was good; there was no, or a relatively low, wash-out of WBCs from the filter **960** by the storage solution (SAG-M); and the overall RBC recovery was very high. This last point appears to be a very important advantage; namely, good RBC filtration with very low RBC loss. Another point to be emphasized is the time gained for operators. By performing high-crit filtration immediately the resulting RBC product units are ready to be stored right from the machine without further processing. Operator time is then freed up for performance of other procedures.

While one preferred approach for RBC collection and filtration has been described above, other approaches will be apparent as well. See, for example, Fig. 4A, wherein an alternative placement of the respective filter **960a** is shown (in dashed lines) relative to a blood component separation device 6. A further description of this and other alternatives are described below.

Though the following are not in any way intended to limit the present invention, Examples A-E are provided to highlight the efficacy hereof.

EXAMPLE A: TABLES 1-3 AND FIG. 5A

A multi-center trial was set up to evaluate the performance of the herein described leukodepletion protocol. The methods generally involved filtration at a high hematocrit started during a continuous apheresis separation and collection process. SAG-M storage solution was added after filtration through the RBC filter. Hematocrits and hemoglobin of the filtered RBCs were measured. Deleukocytation (also known as leukodepletion or leukoreduction) was determined by Nageotte. The results of 147 procedures showed that hematocrit and hemoglobin content were normal (57.3 $\pm$ 3.0 %; 55.1 $\pm$ 4.3 g/unit). All products showed excellent leukodepletion ($\leq$ 0.75 x $10^6$/unit; 99.31 % < 1 x $10^6$). The conclusion is that immediate, on-line, high hematocrit filtration of red cells collected on a Trima® apheresis system (or the like) results in leukoreduced RBCs which meet the American Association of Blood Banks (AABB) and Council of Europe criteria.

Pre-storage leukoreduction is being used more and more in transfusion medicine. Advantages are well-known and plentiful. It has been established that leukoreduction can reduce the number of non hemolytic febrile transfusion reactions whilst other studies have demonstrated its positive aspects in improving the functional quality of erythrocytes during storage and in decreasing the occurrence of alloimmunization in patients receiving multiple transfusions. Storage studies have further shown that important parameters of metabolism such as intra-erythrocyte ATP and extracellular potassium levels tend to decline more slowly in filtered erythrocyte products which is thought to be linked to the lower levels of contaminating enzymes stemming from lysed leukocytes or platelets in the filtered product. Another important factor in favor of leukodepletion is its reduction of transfusion transmitted disease with especially cytomegalovirus (CMV) being of note.

Currently the level of <1x$10^6$ WBCs per transfusion is applied as a transfusion standard in many countries. In some countries components with less than 5x$10^6$ WBCs per component are officially considered sufficiently leukodepleted. Previously, filtration after storage with a bedside filter tended to be the predominant method but filtration efficacy turned out to be highly variable even after only brief storage periods at 4°C. Pre-storage leukoreduction is getting to be more and more widely used. Most of these pre-storage filtrations however still take place after a certain hold-period (for instance overnight) and it was observed that better deleukocytation results had been obtained when RBCs were filtered at lower temperatures. These limitations can make pre-storage filtration still relatively time consuming and labor intensive. The aim of this study was to evaluate a new filtration approach in which red blood cells from automated blood collection were filtered directly during on-going continuous separation and collection, particularly also at high collection hematocrit. Determination of the residual WBC levels after filtration was the main objective. MATERIALS AND METHODS

Description of the Disposable Assembly and the Collection Procedure:

A Trima® disposable assembly such as assembly **10** (Figs. 1 and 2A) which is particularly useful with a Trima® apheresis system such as system 2 (Figs. 1 and 3) for automated collection of platelets/plasma/red cells with a pre-attached leukoreduction filter **960** and filtration sub-assembly **950** was used (Figs. 2A and 2B). RBCs were collected on the Trima® apheresis system at an 80 % target hematocrit. Prior to the collection of the RBCs, the fluid pathway to the filter **960** was opened, allowing immediate filtration from the first milliliter collected in bag **954** onwards. At the end of the filtration 100 ml SAG-M storage solution was added to the RBCs through the filter 960 thus washing out most of the RBCs retained in the filter **960**. During the filtration process air was allowed to enter the filter **960**. This was to occur during each procedure at the end of filtration before addition of the SAG-M storage solution but also frequently (at least 76 % of the runs in this example) at the beginning of RBC collection when the accumulated RBC volume in the intermediate bag **954** (Fig. 2B) is filtered before the apheresis system **2** returned to the collection submode.

During the study care was taken to keep data gathering and analysis well controlled. However, all throughout the study period every participating center retained full freedom in use of its apheresis equipment. This was done intentionally so that the study conditions would be as close as possible to actual routine automated blood collection conditions. The reported data therefore give a good view on how the new high hematocrit filtration protocol performs in a routine setting.

Study design:

A multi-center trial was set up with 3 blood centers. Per center a total of between 35 and 70 procedures was targeted. In Centers coded A and C, all procedures were routine automated platelet and red blood cell collections, whilst in Center B plasma was collected as well. All centers used the same disposable assemblies **10** with an integrated filter **960** and all apheresis procedures were performed according to local and European regulations. With regard to product yield, Centers A and B targeted collection of 180 ml of RBC in 225 ml collect volume and Center C targeted 200 ml RBC in 250 ml. A summary and overview of the study characteristics are given in Table 1, below.

TABLE 1: PARTICIPATING CENTERS AND TYPES OF PROCEDURE

| CENTER | CODE | NUMBER OF PROCEDURES | PROCEDURE TYPE |
|---|---|---|---|
| Ospedale San Bortolo, Vicenza | A | 58 | Platelet-RBC |
| Centro de Transfusion, Madrid | B | 54 | Platelet-RBC-Plasma |
| Universitätsklinikum, Göttingen | C | 35 | Platelet-RBC |

Laboratory analysis

All filtered products were weighed individually. The hematocrit of each filtered product after addition of SAG-M was determined using one of three automated cell counters (Coulter EPICS-XL MCL, Sysmex SE900, Sysmex CS). Residual WBC levels were measured using Nageotte counting in 2 centers. Samples were diluted 1 to 10 in Leucoplate (Plaxan) and one grid (40 lanes, 50 μl diluted sample) was counted. One cell observed in one grid of the Nageotte chamber corresponds to 0.2 WBCs per μl. When no WBCs were found, calculations were performed as if 1 WBC was seen. This prevents the final results from being biased toward lower than real contamination and allows logarithmic analysis. Center B used flowcytometry instead, whereby results exceeding 1 cell/μl were double-checked by means of Nageotte. In Center A in addition to the Nageotte counting described above, residual WBCs were also counted using the Terasaki method for the purpose of comparison. Only the data from Nageotte counting were used in the calculations.

Statistical Analysis

The results of the residual WBC counting were analyzed after $\log_{10}$ transformation. As can be seen in the lognormal probability distribution plot (Fig. 5A), many of the observations at or below the minimum detectable level (± 60 000 WBCs) corresponding to a concentration of 0.2 WBCs per μl result in a deviation from the straight line. These results were included in the plot but were excluded from the least-squares fit of the regression line in order not to bias the prediction.

RESULTS

General

A total of 147 procedures had been performed in the 3 participating centers (58, 54 and 35 procedures in Centers A, B and C respectively). All filtrations completed without any specific side events noted.

Blood Cell Count

Hematocrit (Hct) and hemoglobin (Hgb) content of the filtered products after SAG-M addition were found to comply with the Council of Europe guidelines : 57.3 ± 3.0 % and 55.1 ± 4.3 g/unit respectively. These data are represented in Table 2, below.

Yield

Yield was calculated on the basis of the above hematocrit data and the product volume determined after each filtration. The average efficiency, i.e. the percentage comparing how much actual product was obtained relative to the amount targeted by the machine, was 91.6 ± 4.3 %. Breakdown of the efficiency per center is listed in Table 2, below.

TABLE 2 : PRODUCT CHARACTERISTICS

| CENTER | Hct (%) | Hgb (G/UNIT) | EFFICIENCY (%) |
|---|---|---|---|
| **A** | 55.5 ± 2.6 | 54.2 ± 2.8 | 90.7 ± 4.3 |

(continued)

| CENTER | Hct (%) | Hgb (G/UNIT) | EFFICIENCY (%) |
|---|---|---|---|
| **B** | 58.0 ± 2.8 | 52.8 ± 3.5 | 93.0 ± 4.6 |
| **C** | 59.1 ± 1.9 | 60.5 ± 3.2 | 90.9 ± 3.3 |

White cells

Figure 5A shows the log normal probability plot of the total residual white cell contamination of all procedures. The extrapolated straight line has been obtained by performing a least-squares fit of all data above the detection limit for counting 40 lanes (1 grid) of the Nageotte chamber (0.06 x $10^6$). The population is clearly lognormally distributed with a mean of 0.078 x $10^6$ WBCs per filtered unit (mean ± SD, 4.89 ± 0.45). All products showed good leukodepletion: none of the products contained more than 1x$10^6$ WBCs. In 76 out of 105 performed Nageotte counts (72 %) either one or no WBCs at all were seen in the 40 lanes of the Nageotte chamber which explains the deviation from a straight line below this detection limit. Regression analysis of the linear part of the lognormal probability plot indicates that 99.3 % of the procedures can be expected to contain < 1 x $10^6$ WBC. The median residual WBC level was 0.06 x $10^6$ per product with a minimum of 0.03 x $10^6$ and a maximum of 0.75 x $10^6$ WBCs. Table 3, below, details the break-down for each individual center.

TABLE 3 : RESIDUAL WBCs FOR THE DIFFERENT PARTICIPATING CENTERS

| CENTER | MEDIAN (x$10^6$) | MAX (x$10^6$) | MIN (x$10^6$) | PERCENTAGE WITH < $10^5$ |
|---|---|---|---|---|
| **A** | 0.06 | 0.12 | 0.03 | 88 |
| **B** | 0.15 | 0.75 | 0.03 | 26 |
| **C** | 0.06 | 0.46 | 0.06 | 54 |
| **A+B+C** | 0.06 | 0.75 | 0.03 | 57 |

DISCUSSION

The quantitative aspects of the RBC components obtained after automated blood collection and filtration at high hematocrit are now described. SAG-M storage solution is only added after filtration is finished and is added via the deleukocytation (also known as leukocyte reduction or leukoreduction) filter thus rinsing out part of the RBCs remaining in the filter, tubing lines or the collection bag. Especially the latter might seem to run somewhat contrary to common recognized principles of filtration given the expected risk of washing out the captured WBCs in this way. The data in this study show however that this is not the case. Starting filtration during the automated blood collection results in a finished deleukocytised RBC product shortly after the apheresis procedure is completed.

The results in this study show very good deleukocytation characteristics and demonstrate that filtration at high hematocrit offers an efficient and reproducible way of leukodepleting RBC products. Residual WBCs in all filtered products remained well below the 1 x $10^6$ limit per unit and more than half of the products were even found to contain less than 1 x $10^5$ WBCs per unit. Efficiency, expressed as the percentage of the measured yield over the targeted yield, was very good and resulted in more than 90 % recovery of the RBC product. The recovery reported here is most probably underestimated. Since the prefiltration product never actually exists as a whole product (a part is already filtered while the product is still being collected), no accurate prefiltration dose can be established. The dose targeted by the Trima® apheresis system was used in the recovery calculations as prefiltration dose. Earlier observations in Europe have shown that actual collected doses by the Trima® apheresis system tend to be a few percent below targeted dose as expressed in absolute volume of red cells. This might be related to differences between hematocrits obtained through centrifuged methodology versus impedance automatic counters. Furthermore, the hyperosmolality of SAG-M might induce some shrinkage of RBCs. In another study where more careful attempts were used to estimate the pre-filtration dose, recoveries were found to be around 97%. There were no signs of wash out of the trapped leukocytes. Other studies have demonstrated normal storage of RBCs using the same filtration and collection approach.

In conclusion, the evaluated high hematocrit filtration protocol has proven to be a reliable and efficient WBC reduction system allowing centers to leukodeplete RBC products in a systematic and fast manner whilst still retaining high quality results. It complements the versatility of the automated blood collection process in that products at the end of the procedure no longer require further processing.

EXAMPLE B: INSTRUCTIONS FOR USE

Set forth here are more details concerning the preferred procedures to be used in Example A, above, and Examples C-E, except where otherwise noted. Specific reference to the Trima® Automated Blood Component Collection System Operator's Manual or the herein-above listed patent publications is suggested for further specifics regarding the following procedures: setting up the disposable tubing set, performing the collection procedure and, removing the disposable set.

With specific reference to Figs. 2B, 3 and 4A-4B, the order of preferred steps is as follows:

1. Break the frangible connector **967** above the filter **960** (if such an optional frangible connector is included in the assembly **10**) prior to the start of the apheresis procedure or, if platelets are also to be collected herein, then during the platelet collection phase prior to the start of the RBC collection phase.

2. Close the air removal clamp **963** near the RBC collection bag **958**.

3. Ensure that the clamp **966** between the filter **960** and the collection bag **958** is open.

4. Hang the intermediate bag **954** on the I.V. pole hook **980**. Hang the collection bag **958** and additional tubing line on the lower hook **981** located on the side of the apheresis machine **6**. Ensure that the RBC flow to the collection bag **958** is not impeded. Smooth out any kinks in the tubing line.

5. Properly label the collection bag **958**.

6. RBC filtration is preferred to occur at room temperature.

7. Once the RBC collection phase has started and the RBCs have entered the filter **960**, it is acceptable for air to enter the enter inlet side of the filter **960**. Minimize any external physical forces on the RBC intermediate bag **954** and/or the filter **960** or any sudden flow changes. Do not squeeze the RBC intermediate bag **954** to increase flow rates during this step.

8. Filtration is complete when the inlet side of the filter housing **960** is empty.

9. Remove intermediate bag **954** from the apheresis machine **6** pole hook **980** and hang it on the middle hook **982** located on the side of the apheresis machine **6**.

10. Hang the additive solution (AS-3, SAG-M) on the I.V. pole hook **980** and spike connect the additive solution bag **970** to the intermediate bag **954** which contained, but is now preferably devoid of, RBCs.

11. Break the frangible connector **968** between the intermediate bag **954** and the spike **959** and allow the additive solution to transfer through the intermediate bag **954** and the filter **960** into the collection bag **958**.

12. Filtration flushing is compete when the inlet side of the filter housing is empty.

13. Seal the outlet tubing line **965** next to the outlet port of the filter **960**. Discard the intermediate bag **954**, the filter **960** and the outlet clamp **966** per standard operating procedures. Weigh the collection bag **958** (include the tubing line **961** and air removal bag **962** that remain connected).

14. Strip the tubing line **965** containing the additive solution into the collection bag **958** preferably three times. If necessary, the emptied line can be sealed off partly when there is no need for all of the available segments.

15. After stripping, do not mix the RBCs as this may cause foam formation and may cause difficulty for later air removal. Mixing should be performed after air removal.

16. For air removal, hold the collection bag **958** vertically with ports up. Open the air removal clamp **963** and squeeze air from the collection bag **958** into the air removal bag **962**. Close the air removal clamp **963**. Do not express RBCs into the air removal tubing line **961**.

17. Mix the RBCs in the collection bag **958** thoroughly.

18. For quality control and/or retention segment sampling, open the air removal clamp **963** and express the RBCs into the air removal tubing line **961**. Close the clamp **963**.

19. Seal the segmented inlet tubing line **965** of the collection bag **958** and the air removal tubing line **961** per standard operating procedures.

20. Discard the air removal bag **962** and the air removal clamp **963**.

EXAMPLE C: TABLE 4 AND FIGURE 5B

The goal in this example was to try filtering simultaneously with the collection of red blood cells. After a small amount of high-crit red cells had been intermediately collected, the frangible connection **967** was broken and the red cells were allowed to flow through the filter **960** into the second bag **958**. Then, storage solution was added through the filter **960** according to the above-detailed procedures. Samples for Nageotte counts were taken from the high-crit filtered red cells before storage solution was added and again after the addition of storage solution, and from the segment just below the filter after the storage solution was added. Single and double red blood cell (DRBC) units were collected and used in this example. Fig. 5B is the log-normal plot for these results. The count increases after the storage solution is added through the filter which may imply that the storage solution is washing some cells off the filter.

TABLE 4.

| | Actual Volume | Pre Storage Solution calc'd using 255 volume | | Pre SS calc'd w/ Actual Volumes | Post Storage Solution | | Post SS calc'd w/ Actual Volumes | Segment Post SS | | Donor Pre-Count |
|---|---|---|---|---|---|---|---|---|---|---|
| **Sample Id** | Pre SS | cells/ul | cells/unit x10^6 | cells/unit x10^6 | cells/ul | cells/unit x10^6 | cells/unit x10^6 | cells/ul | cells/unit x10^6 | x10^3/ul |
| RBCPLTHB1-RBC | 251.0 | 0.8 | 0.20 | 0.20 | 1.6 | 0.57 | 0.56 | 0.4 | 0.14 | 6.00 |
| DRBCHB8-RBCA | 240.0 | 0.6 | 0.15 | 0.14 | 1 | 0.36 | 0.34 | 0.1 | 0.04 | 6.20 |
| DRBCHB8-RBCB | 261.5 | 0.6 | 0.15 | 0.16 | 1.2 | 0.43 | 0.43 | 0.1 | 0.04 | 6.20 |
| DRBCHB9-RBCA | 255.8 | 0.4 | 0.10 | 0.10 | 0.8 | 0.28 | 0.28 | 0.1 | 0.04 | 4.80 |
| DRBCHB9-RBCB | 245.3 | 0.6 | 0.15 | 0.15 | 0.8 | 0.28 | 0.28 | 0.1 | 0.04 | 4.80 |

Example of calculation for RBCPLTHB1-RBC - Hi-crit

$$0.8\,\frac{\text{Cells}}{\mu\ell} \times \frac{1\,\mu\ell}{10^{-6}\,\ell} \times \frac{1\,\ell}{1000\text{ ml}} \times 251\text{ ml} = 0.2\,\frac{\text{cells}}{\text{unit}} \times 10^{6}$$

Example D: Table 5 and Figures 5C and 5D

The data shown in Table 5 were generated by a) sampling the high-crit cells before storage solution was added; b) sampling the storage solution that was flushed through the filter and collected in a separate bag; and c) sampling the high-crit cells after fresh storage solution was added. In these samplings, actual volumes were used to calculate the number of cells per unit.

Figure 5C graphs the high-crit before and after the addition of storage solution. In this example, the high-crit cell population and the high-crit and storage solution population are more inter-mixed when using actual volumes. There were not enough samples with values above 60,000/unit for the storage solution flush data to appear on the graph, but it appears that the cells have been washed off.

Figure 5D is a graph of the same data as shown in Figure 5C removing the high data point in the high-crit population. With the deletion of this data point, the variance in the high-crit count before and after storage solution is added is very small. The pre-storage solution population is the same as the post-storage solution population as they ought to be.

TABLE 5.

| STORAGE SOLUTION FLUSH THROUGH FILTER INTO SEPARATE BAG CLEAN STORAGE SOLUTION ADDED TO CELLS | | | | | | | | | | | | |
|---|---|---|---|---|---|---|---|---|---|---|---|---|
| | **Actual Volume** | **Pre Storage Solution calc'd using 255 volume** | | **Pre SS calc'd w/ Actual Volumes** | **Storage Solution Flush into separate bag** | | **Post Storage Solution Clean Storage Solution** | | **Post SS calc'd w/ Actual Volumes** | **Pre SS + SS Flush** | **Post SS + SS Flush** | **Donor Pre Count** |
| **Sample Id** | Pre SS | cells/ul | cells/unit x10^6 | cells/unit x10^6 | cells/ul | cells/unit x10^6 | cells/ul | cells/unit x10^6 | cells/unit x10^6 | cells/unit x10^6 | cells/unit x10^6 | x10^3/ul |
| DRBCHB10-RBCA | 253.1 | 0.4 | 0.10 | 0.10 | 0.2 | 0.02 | 0.4 | 0.14 | 0.14 | 0.12 | 0.16 | 6.70 |
| DRBCHB10-RBCB | 247.9 | 0.2 | 0.05 | 0.05 | 0.2 | 0.02 | 0.2 | 0.07 | 0.07 | 0.07 | 0.09 | 6.70 |
| DRBCHB13-RBCA | 256.8 | 10.2 | 2.60 | 2.62 | 0.8 | 0.08 | 1.2 | 0.43 | 0.43 | 2.70 | 0.51 | 4.90 |
| DRBCHB13-RBCB | 244.3 | 2 | 0.51 | 0.49 | 0.4 | 0.04 | 1.2 | 0.43 | 0.41 | 0.53 | 0.45 | 4.90 |
| DRBCHB14-RBCA | 250.9 | 1.4 | 0.36 | 0.35 | 0.2 | 0.02 | 0.2 | 0.07 | 0.07 | 0.37 | 0.09 | 5.80 |
| DRBCHB14-RBCB | 249.1 | 0.6 | 0.15 | 0.15 | 0.2 | 0.02 | 0.4 | 0.14 | 0.14 | 0.17 | 0.16 | 5.80 |
| RBCPLAHB2-RBC | 251.0 | 0.8 | 0.20 | 0.20 | 0.1 | 0.01 | 0.8 | 0.28 | 0.28 | 0.21 | 0.29 | 3.60 |
| RBCPLAHB3RBC | 251.0 | 0.2 | 0.05 | 0.05 | 0.1 | 0.01 | 0.2 | 0.07 | 0.07 | 0.06 | 0.08 | 5.50 |
| DRBCHB15-RBCA | 253.3 | 0.8 | 0.20 | 0.20 | 0.2 | 0.02 | 0.8 | 0.28 | 0.28 | 0.22 | 0.30 | 6.50 |
| DRBCHB15-RBCB | 247.8 | 1 | 0.26 | 0.25 | 0.4 | 0.04 | 0.8 | 0.28 | 0.28 | 0.29 | 0.32 | 6.50 |
| DRBCHB16-RBCA | 243.3 | 0.6 | 0.15 | 0.15 | 0.1 | 0.01 | 0.4 | 0.14 | 0.14 | 0.16 | 0.15 | 6.60 |
| DRBCHB16-RBCB | 257.9 | 0.4 | 0.10 | 0.10 | 0.1 | 0.01 | 0.2 | 0.07 | 0.07 | 0.11 | 0.08 | 6.60 |

Example E: Table 6, Figs, 5E, 5F and 5G

These data were collected with the same procedure as the preceding examples, but without diluting the high-crit cells with storage solution before counting them. Each plot contains multiple data sets; one with a high-crit before dilution with storage solution, one with the addition of storage solution via a storage solution flush, and one with a further addition of clean storage solution not flushed through the filter. The concentrations calculated with actual volumes were graphed.

Figure 5E is a regression of storage solution flush data above 61,000. Note that the regression shows a greater difference between the high-crit without storage solution and the high-crit with storage solution than the above counting methods.

Figure 5F is the same regression of storage solution flush data as shown in Figure 5E with the addition of two additional filtration samples. The addition of the additional data helps adjust for the skew caused by a single filter failure sample, greatly improving the probabilities.

Figure 5G includes the same data as Figure 5F with the addition of data from the white cells from the storage solution flush added to the data for the cells for the high-crit cells and storage solution. The cells/unit ratio was calculated based on the volume of the high-crit storage solution volume. Theoretically, this figure is the number of cells that would have been in the filtered product if the storage solution were flushed through the filter without the filter failure.

TABLE 6.

| STORAGE SOLUTION FLUSH THROUGH FILTER INTO SEPARATE BAG CLEAN STORAGE SOLUTION ADDED TO CELLS, BUT STORAGE SOLUTION ADDED TO HI-CRIT SAMPLES BEFORE COUNTING | | | | | | | | | | | | |
|---|---|---|---|---|---|---|---|---|---|---|---|---|
| | **Actual Volume** | **Pre Storage Solution calc'd using 255 volume** | | **Pre SS calc'd w/ Actual Volumes** | **Storage Solution Flush into separate bag** | | **Post Storage Solution Clean Storage Solution** | | **Post SS calc'd w/ Actual Volumes** | **Pre SS + SS Flush** | **Post SS + SS Flush** | **Donor Pre Count** |
| **Sample Id** | Pre SS | cells/ul | cells/unit x10^6 | cells/unit x10^6 | cells/ul | cells/unit x10^6 | cells/ul | cells/unit x10^6 | cells/unit x10^6 | cells/unit x10^6 | cells/unit x10^6 | x10^3/ul |
| DHBCHB17-RBCA | 254 02 | 1.5 | 0.38 | 0.38 | 0.7 | 0.07 | 0.6 | 0.21 | 0.21 | 0.45 | 0.28 | 6.9 |
| DRBCHB17-RBCB | 247.03 | 1.2 | 0.31 | 0.30 | 1.2 | 0.12 | 0.4 | 0.14 | 0.14 | 0.42 | 0.26 | 6.9 |
| RBCPLAHB2 | 250.00 | 0.9 | 0.23 | 0.23 | 0.2 | 0.02 | 02 | 0.07 | 0.07 | 0.25 | 0.09 | 6.2 |
| DRBCHB18-RBCA | 265.39 | 1.5 | 0.38 | 0.40 | 1.3 | 0.13 | 0.8 | 0.28 | 0.29 | 0.53 | 0.42 | 4.9 |
| DRBCHB18-RBCB | 236.45 | 1.2 | 0.31 | 0.28 | 1.2 | 0.12 | 0.6 | 0.21 | 0.20 | 0.40 | 0.32 | 4.9 |
| DRBCHB19-RBCA | 209.86 | 1.5 | 0.38 | 0.31 | 1.3 | 0.13 | 1.4 | 0.50 | 0.43 | 0.44 | 0.56 | 5.2 |
| DRBCHB19-RBCB | 209.86 | 9.6 | 2.45 | 2.01 | 6.6 | 0.66 | 5.4 | 1.92 | 1.67 | 2.67 | 2.33 | 5.2 |
| RBCPLTHB4 | 251.00 | 1.2 | 0.31 | 0.30 | 0.2 | 002 | 0.2 | 0.07 | 0.07 | 0.32 | 0.09 | 5.2 |
| RBCPLTHB5 | 251.00 | 1.2 | 0.31 | 0.30 | 0.9 | 0.09 | 0.2 | 0.07 | 0.07 | 0.39 | 0.16 | 6.5 |
| RBCPLTHB6 | 251.00 | 0.6 | 0.15 | 0.15 | 0.2 | 0.02 | 0.8 | 0.28 | 0.28 | 0.17 | 0.30 | 4.8 |

Numerous further alternative elements and/or embodiments are available. For example, in order to assist an operator in performing the various steps of the protocol being used in an apheresis procedure with the apheresis system **2**, the apheresis system 2 preferably includes a computer graphical interface **660** as illustrated generally in Fig. 1. The graphical interface **660** may preferably include a computer display **664** which has "touch screen" capabilities; however, other appropriate input devices (*e.g.*, keyboard) may also be utilized alone or in combination with the touch screen. The graphics interface **660** may provide a number of advantages, but may preferably, at least, assist the operator by providing pictorials of how and/or when the operator may accomplish at least certain steps of the apheresis and/or filtration procedures.

For example, the display screen may sequentially display a number of pictorials to the operator to convey the steps which should be completed to accomplish the filtering procedure described here. More particularly, a pictorial image may be shown on the screen to pictorially convey to the operator when and/or how to hang the respective RBC bags **954** and/or **958** on the machine **6**, initially and/or during a subsequent storage solution flush (see Figs. 3, and Figs. 4A and 4B, for example). One or more pictorials may also be provided to instruct the operator when to break the frangible connector **967** (if included) to begin the filtration process, and/or to visually ensure that the filtration process has appropriately begun simultaneously or during RBC collection. One or more pictorials may also be used to instruct the operator when to connect the spike assembly **956** to a storage solution container **970** and/or when to break the frangible connector **968** (if included) after the RBCs have run through filter **960**, to then run the storage solution through the filter **960** and flush any residual RBCs therethrough. One or more pictorials may also be used to instruct the operator when the tube line **952** leading to the intermediate RBC bag **954** should be sealed such that the RBC collect bag **954**, and the remaining elements of filter storage assembly **950** may be separated and/or removed from the disposable assembly **10** and/or from the device **6**. A similar pictorial can instruct when to seal the tube **965** and/or tube **966** to isolate the RBC collection bag **958** from the rest of the system after the filtration and flushing procedures are completed.

A further advantage of the presently described system includes the manner of handling air. More specifically, the present invention eliminates the prior need for the vents and/or by-pass methods and/or apparatuses of conventional red blood cell filters. Moreover, the present invention is capable of delivering this advantage with no reduction in and/or perhaps an increase in the recovery of RBCs that historically have been trapped inside the filtration device.

A means used by the present invention to deliver this advantage is through the providing of a storage solution flush through the filter after the RBCs have finished filtering therethrough. The storage solution may then be able to wash RBCs caught therein out of the filter and then into the collection bag **958**. Prior devices relied upon vents or by-pass mechanisms to assist in pushing out any RBCs disposed in the filter. Note, though not preferred or needed, vents or by-passes could still be used with the high hematocrit filtration process, and also with and/or in lieu of the storage solution flush after filtration.

In any event, elimination of the need for vents or by-passes also reduces other prior difficulties such as inadvertent allowances of excess air into the system or the requiring of certain predefined lengths of tubing lines on respective sides of the filter. Extra air in the present system will not stop or slow the flow of blood or storage solution through the filter in the present invention. The extra air will either be caught within the intermediate bag **954** or pass through to the collection bag **958** where it can be removed at the end of the overall process to the air bag **962**. Then, also, because neither vents nor by-passes are used or needed in the preferred embodiments here, the tubing line lengths important to many prior devices and methods, are not so significant here. Hydrostatic pressures caused by the respective heights of the fluids contained within certain tubing line lengths can counteract the operation of vents; however, this is not problematic here since the preferred subsequent storage solution flush recovers the RBCs from the filter without the previously desired use of a vent or by-pass. Consequently, also, the filter may be disposed at any of a plurality of alternative vertical dispositions between intermediate bag **954** and collection bag **958**; see, for example, the dotted line alternative filter marked **960a** in Fig. 4A. Operation of the present invention is not hindered by such alternative placements.

The volume of storage solution to be used may, however, be modified depending upon the relative lengths of tubing lines used and/or the air that gets into the system. Thus, if it is known that there is 20-30 ml of dead space in the filter and, say, approximately 20 ml of tubing line between intermediate bag **954** and collection bag **958**; and if 100 ml of storage solution is desired to be mixed with the end product RBCs in collection bag **958**; then some certain volume more than 100 ml of storage solution would preferably be fed into the system. For example, 140-150 ml would preferably be added; whereby 100 ml of which would go into the collection bag **958** and the remaining 40-50 ml would fill the dead spaces in the tubing line and filter between intermediate bag **954** and collection bag **958**.

Note, although a storage solution flush after filtration completion is preferred, alternatives are available here as well. For example, though not preferred, it is possible that storage solution flow into bag **954** may be begun prior to absolute completion of the high-crit RBC filtration. Thus, whatever quantity of RBCs remaining in bag **954** at this point would then be diluted by the storage solution prior to filtration hereof. This is not preferred because it may be that such a diluted end remainder of RBCs might contribute to washing out some WBCs caught in the filter **960**.

Other storage solution alternatives include not flushing the storage solution through the filter **960** at all. Such storage solution may be added in other ways; for example by being resident in the ultimate collection bag **958** prior to the inflow of filtered high-crit RBCs thereinto. Or, the storage solution could be flowed past (by-pass) filter **960** directly into the collection bag **958** during or after the flow of filtered RBCs thereinto.

Note, in the currently described invention, the gravity flow rate out of bag **954** is not very different from the flow rates of RBCs entering intermediate bag **954** from the centrifuge. Thus, a smaller intermediate bag **954** is foreseeably useful herewith as well. By way of example, a intermediate bag **954** could practically be half, or less, than the size of a standard collection bag **958**, under present operating conditions.

Alternative extracorporeal blood processing systems may be used to provide the red blood cells. Although the preference is for a continuous flow apheresis system, as described here, which includes returning some components back to the donor, batch flow and non-return systems are also useable herewith. For example, a batch mode processor takes in a certain quantity of whole blood, separates the blood into components (in a centrifuge bowl, *e.g.*) and then passes the separated components to collection containers or back to the donor. The filtration process of the present invention would nevertheless operate in substantially the same manner such that the separated RBCs would nonetheless exist in a substantially high hematocrit state as they are flowed from the separation mechanism, at which point these high-crit separated RBCs could be flowed to an intermediate bag **954** (Figs. 2B and 3, *inter alia*), and from there be passed directly or soon thereafter to and through a filter **960** to be collected ultimately in a collection bag **958**. Though continuity may be reduced (or substantially removed), the principles of filtration remain the same. Note, even if flow through the filter **960** stops at any point, or a plurality of points, this is not problematic here where any air entry therein is handled by capture in the air bag **962**.

Smaller scale separation and collection devices are also envisioned to be useful herewith. For example, various separation devices (whether centrifugal or membrane or other types) are designed to separate only RBCs and plasma (with the remainder usually remaining in the RBC product), and these can take on smaller scale mechanizations. Nevertheless, the present invention is useful herewith as well in that RBCs separated hereby may also be freshly filtered at high, undiluted hematocrits. The principle of filtering such RBCs during or soon after the overall separation and collection process remains the same here as well. Thus, whether continuous or in batch mode, a flow of high-crit, freshly-separated RBCs can be flowed from the separation device to an intermediate bag **954** and from there immediately or soon after accumulation therein, to and through filter **960** to collection bag **958**.

The foregoing description of the present invention has been presented for purposes of illustration and description. Furthermore, the description is not intended to limit the invention to the form disclosed herein. Consequently, variations and modifications commensurate with the above teachings, and skill and knowledge of the relevant art, are within the scope of the present invention. The embodiments described hereinabove are further intended to explain best modes known of practicing the invention and to enable others skilled in the art to utilize the invention in such, or other embodiments and with various modifications required by the particular application(s) or use(s) of the present invention.

## Claims

**1.** A method for the leukoreduction of red blood cells comprising:

providing freshly-separated high hematocrit red blood cells recently-removed from a donor;
passing said freshly-separated high hematocrit red blood cells through a leukoreduction filter (960);
collecting the high hematocrit red blood cells in a collection container (958) after said step of passing said high hematocrit red blood cells through said leukoreduction filter (960);
flowing a storage solution through said leukoreduction filter (960) after said passing step; and
collecting said storage solution in said collection container (958) for mixing with said high hematocrit red blood cells.

**2.** A method according to claim 1 in which said high hematocrit red blood cells have a hematocrit of between about 70 and about 90.

**3.** A method according to claim 1 in which said high hematocrit red blood cells have a hematocrit of about 80.

**4.** A method according to claim 1 in which said high hematocrit red blood cells have a hematocrit between 80 and 95+.

**5.** A method according to claim 1 which further comprises a step of passing the freshly-separated high hematocrit red blood cells to an intermediate red blood cell container prior to said step of passing the high hematocrit red blood cells through the leukoreduction filter.

**6.** A method according to claim 1 in which said step of passing the freshly-separated high hematocrit red blood cells through the leukoreduction filter occurs substantially simultaneously with said step of providing freshly-separated high hematocrit red blood cells.

**7.** A method according to claim 1 in which said step of passing the freshly-separated high hematocrit red blood cells through the leukoreduction filter occurs soon after said step of providing freshly-separated high hematocrit red blood cells.

**8.** A method according to claim 1 in which said passing step occurs within zero (0) to two (2) hours of said providing step.

**9.** A method according to claim 1 in which said passing step occurs within zero (0) to four (4) hours of said providing step.

**10.** A method according to claim 1 in which said passing step occurs within zero (0) to eight (8) hours of said providing step.

**11.** A method according to claim 1 in which said passing step occurs within zero (0) to twenty-four (24) hours of said providing step.

**12.** A method according to claim 1 in which said step of providing freshly-separated high hematocrit red blood cells further comprises:

separating the red blood cells from other blood components using a separation system to produce freshly separated high hematocrit red blood cells.

**13.** A method according to claim 12 in which said separation system is an apheresis system.

**14.** A method according to claim 12 in which said separation system is a centrifugal separation system.

**15.** A method according to claim 12 in which said separation system is membrane-based.

**16.** A method according to claim 12 in which said separation system is a substantially continuous flow system.

**17.** A method according to claim 12 in which said system is a batch-mode system.

**18.** A method according to claim 12, wherein said leukoreduction filter and red blood cell collection container are comprised in a red blood cell collection assembly.

**19.** A method according to claim 18 in which said red blood cell collection assembly further comprises an intermediate red blood cell collection reservoir which is interposed between said separation system and said leukoreduction filter; the method further comprising:

passing the high-hematocrit separated red blood cells from said separation system into the intermediate red blood cell reservoir prior to said step of passing the high-hematocrit separated red blood cells through said leukoreduction filter.

**20.** A method according to claim 18 in which said red blood cell collection assembly further comprises a sterile barrier/spike assembly for connection of said red blood cell collection assembly to a source of storage solution; said sterile barrier/spike assembly being interposed between said separation system and said leukoreduction filter; the method further comprising
connecting said sterile barrier/spike assembly to a source of storage solution; and
adding a storage solution to the high-hematocrit separated red blood cells after said step of passing said high-hematocrit separated red blood cells through said leukoreduction filter.

**21.** A method according to claim 19 in which said red blood cell collection assembly further comprises a sterile barrier/spike assembly for connection of said red blood cell collection assembly to a source of storage solution; said sterile barrier/spike assembly being connected to said intermediate red blood cell reservoir; the method further comprising connecting said sterile barrier/spike assembly to a source of storage solution; and
passing a storage solution to the intermediate red blood cell reservoir and then to and through said leukoreduction filter and to the collection container to mix with the separated red blood cells after said step of passing said separated red blood cells.

**22.** A method according to claim 19 in which said intermediate red blood cell reservoir is connected to said leukoreduction filter by a frangible connector; whereby said frangible connector blocks fluid flow therethrough; the method further comprising selectively opening said frangible connector to allow flow therethrough.

**23.** A method according to claim 18 in which the red blood cell collection container is a first red blood cell collection reservoir and wherein said red blood cell collection assembly further comprises a second red blood cell collection reservoir which is disposed after said leukoreduction filter; the method further comprising:

passing the high-hematocrit separated red blood cells from said separation system into and through said leukoreduction filter simultaneously into the first and second red blood cell collection reservoirs.

**24.** A method according to claim 18 in which the red blood cell collection container is a first red blood cell collection reservoir and wherein said red blood cell collection assembly further comprises a second red blood cell collection reservoir which is disposed after said leukoreduction filter; the method further comprising:

passing the high-hematocrit separated red blood cells from said separation system into and through said leukoreduction filter firstly into one of the first and second red blood cell collection reservoirs, and then secondly into the other of said first and second red blood cell collection reservoirs.

**Patentansprüche**

**1.** Verfahren zur Leukoreduktion von roten Blutkörperchen, umfassend:

das Bereitstellen von frisch abgetrennten roten Blutkörperchen mit hohem Hämatokritwert, welche kürzlich von einem Spender entnommen wurden;
das Passieren der frisch abgetrennten roten Blutkörperchen mit hohem Hämatokritwert durch ein Leukoreduktionsfilter (960);
das Sammeln der roten Blutkörperchen mit hohem Hämatokritwert in einem Sammelbehälter (958) nach dem Schritt des Passierens der roten Blutkörperchen mit hohem Hämatokritwert durch das Leukoreduktionsfilter (960);
das Fließen einer Vorratslösung durch das Leukoreduktionsfilter (960) nach dem Passierschritt; und
das Sammeln der Vorratslösung in dem Sammelbehälter (958) zum Mischen mit den roten Blutkörperchen mit hohem Hämatokritwert.

**2.** Verfahren gemäß Anspruch 1, wobei die roten Blutkörperchen mit hohem Hämatokritwert einen Hämatokritwert von zwischen etwa 70 und etwa 90 aufweisen.

**3.** Verfahren gemäß Anspruch 1, wobei die roten Blutkörperchen mit hohem Hämatokritwert einen Hämatokritwert von etwa 80 aufweisen.

**4.** Verfahren gemäß Anspruch 1, wobei die roten Blutkörperchen mit hohem Hämatokritwert einen Hämatokritwert zwischen 80 und 95+ aufweisen.

**5.** Verfahren gemäß Anspruch 1, welches ferner einen Schritt des Passierens der frisch abgetrennten roten Blutkörperchen mit hohem Hämatokritwert in einen rote Blutkörperchen-Zwischenbehälter, vor dem Schritt des Passierens der roten Blutkörperchen mit hohem Hämatokritwert durch das Leukoreduktionsfilter umfasst.

**6.** Verfahren gemäß Anspruch 1, wobei der Schritt des Passierens der frisch abgetrennten roten Blutkörperchen mit hohem Hämatokritwert durch das Leukoreduktionsfilter im Wesentlichen gleichzeitig mit dem Schritt des Bereitstellens von frisch abgetrennten roten Blutkörperchen mit hohem Hämatokritwert stattfindet.

**7.** Verfahren gemäß Anspruch 1, wobei der Schritt des Passierens der frisch abgetrennten roten Blutkörperchen mit hohem Hämatokritwert durch das Leukoreduktionsfilter bald nach dem Schritt des Bereitstellens von frisch abgetrennten roten Blutkörperchen mit hohem Hämatokritwert stattfindet.

**8.** Verfahren gemäß Anspruch 1, wobei der Passierschritt innerhalb von null (0) bis zwei (2) Stunden des Bereitstellungsschritts stattfindet.

**9.** Verfahren gemäß Anspruch 1, wobei der Passierschritt innerhalb von null (0) bis vier (4) Stunden des Bereitstellungsschritts stattfindet.

**10.** Verfahren gemäß Anspruch 1, wobei der Passierschritt innerhalb von null (0) bis acht (8) Stunden des Bereitstellungsschritts stattfindet.

**11.** Verfahren gemäß Anspruch 1, wobei der Passierschritt innerhalb von null (0) bis vierundzwanzig (24) Stunden des Bereitstellungsschritts stattfindet.

**12.** Verfahren gemäß Anspruch 1, wobei der Schritt des Bereitstellens von frisch abgetrennten roten Blutkörperchen mit hohem Hämatokritwert ferner umfasst:

das Abtrennen der roten Blutkörperchen von anderen Blutkomponenten unter Verwendung eines Abtrennungssystems, wobei frisch abgetrennte rote Blutkörperchen mit hohem Hämatokritwert hergestellt werden.

**13.** Verfahren gemäß Anspruch 12, wobei das Abtrennungssystem ein Apheresesystem ist.

**14.** Verfahren gemäß Anspruch 12, wobei das Abtrennungssystem ein Zentrifugations-Abtrennungssystem ist.

**15.** Verfahren gemäß Anspruch 12, wobei das Abtrennungssystem auf einer Membran basiert.

**16.** Verfahren gemäß Anspruch 12, wobei das Abtrennungssystem ein im Wesentlichen kontinuierliches Fluss-System ist.

**17.** Verfahren gemäß Anspruch 12, wobei das System ein Chargen-System ist.

**18.** Verfahren gemäß Anspruch 12, wobei das Leukoreduktionsfilter und der rote Blutkörperchen-Sammelbehälter in einer rote Blutkörperchen-Sammelanordnung umfasst werden.

**19.** Verfahren gemäß Anspruch 18, wobei die rote Blutkörperchen-Sammelanordnung ferner ein rote Blutkörperchen-Sammelzwischenreservoir, welches zwischen dem Abtrennungssystem und dem Leukoreduktionsfilter eingebracht ist, umfasst; wobei das Verfahren ferner umfasst:

das Passieren der abgetrennten roten Blutkörperchen mit hohem Hämatokritwert von dem Abtrennungssystem in das rote Blutkörperchen-Zwischenreservoir, vor dem Schritt des Passierens der abgetrennten roten Blutkörperchen mit hohem Hämatokritwert durch das Leukoreduktionsfilter.

**20.** Verfahren gemäß Anspruch 18, wobei die rote Blutkörperchen-Sammelanordnung ferner eine sterile Barriere/Spike-Anordnung zum Anbinden der rote Blutkörperchen-Sammelanordnung an eine Quelle einer Vorratslösung umfasst; wobei die sterile Barriere/Spike-Anordnung zwischen dem Abtrennungssystem und dem Leukoreduktionsfilter eingebracht ist; wobei das Verfahren ferner umfasst
das Anbinden der sterilen Barriere/Spike-Anordnung an eine Quelle einer Vorratslösung; und
das Geben einer Vorratslösung zu den abgetrennten roten Blutkörperchen mit hohem Hämatokritwert, nach dem Schritt des Passierens der abgetrennten roten Blutkörperchen mit hohem Hämatokritwert durch das Leukoreduktionsfilter.

**21.** Verfahren gemäß Anspruch 19, wobei die rote Blutkörperchen-Sammelanordnung ferner eine sterile Barriere/Spike-Anordnung zum Anbinden der rote Blutkörperchen-Sammelanordnung an eine Quelle einer Vorratslösung umfasst; wobei die sterile Barriere/Spike-Anordnung an das rote Blutkörperchen-Zwischenreservoir angebunden ist; wobei das Verfahren ferner umfasst
das Anbinden der sterilen Barriere/Spike-Anordnung an eine Quelle einer Vorratslösung; und
das Passieren einer Vorratslösung zu dem rote Blutkörperchen-Zwischenreservoir und dann zu und durch das Leukoreduktionsfilter und zu dem Sammelbehälter, um mit den abgetrennten roten Blutkörperchen zu mischen, nach dem Schritt des Passierens der abgetrennten roten Blutkörperchen.

**22.** Verfahren gemäß Anspruch 19, wobei das rote Blutkörperchen-Zwischenreservoir an das Leukoreduktionsfilter durch eine fragile Verbindungsvorrichtung angebunden ist, wobei die fragile Verbindungsvorrichtung einen Fluidfluss dadurch blockiert; wobei das Verfahren ferner ein selektives Öffnen der fragilen Verbindungsvorrichtung, um einen Fluss dadurch zu ermöglichen, umfasst.

**23.** Verfahren gemäß Anspruch 18, wobei der rote Blutkörperchen-Sammelbehälter ein erstes rote Blutkörperchen-Sammelreservoir ist und wobei die rote Blutkörperchen-Sammelanordnung ferner ein zweites rote Blutkörperchen-Sammelreservoir umfasst, welches nach dem Leukoreduktionsfilter angeordnet ist; wobei das Verfahren ferner umfasst:

das Passieren der abgetrennten roten Blutkörperchen mit hohem Hämatokritwert aus dem Abtrennungssystem in und durch das Leukoreduktionsfilter, gleichzeitig in die ersten und zweiten rote Blutkörperchen-Sammelreservoire.

**24.** Verfahren gemäß Anspruch 18, wobei der rote Blutkörperchen-Sammelbehälter ein erstes rote Blutkörperchen-Sammelreservoir ist und wobei die rote Blutkörperchen-Sammelanordnung ferner ein zweites rote Blutkörperchen-Sammelreservoir umfasst, welches nach dem Leukoreduktionsfilter angeordnet ist; wobei das Verfahren ferner umfasst:

das Passieren der abgetrennten roten Blutkörperchen mit hohem Hämatokritwert aus dem Abtrennungssystem in und durch das Leukoreduktionsfilter, erstens in eines der ersten und zweiten rote Blutkörperchen-Sammelreservoire und dann zweitens in das andere der ersten und zweiten rote Blutkörperchen-Sammelreservoire.

**Revendications**

**1.** Procédé pour la leucoréduction de globules rouges comprenant
la fourniture de globules rouges d'hématocrite élevé venant d'être séparés, récemment prélevés d'un donneur ;
le passage desdits globules rouges d'hématocrite élevé venant d'être séparés à travers un filtre de leucoréduction (960) ;
la collecte des globules rouges d'hématocrite élevé dans un conteneur de collecte (958) après ladite étape de passage desdits globules rouges d'hématocrite élevé à travers ledit filtre de leucoréduction (960) ;
l'écoulement d'une solution de stockage à travers ledit filtre de leucoréduction (960) après ladite étape de passage ; et
la collecte de ladite solution de stockage dans ledit conteneur de collecte (958) pour mélange avec lesdits globules rouges d'hématocrite élevé.

**2.** Procédé selon la revendication 1, dans lequel lesdits globules rouges d'hématocrite élevé ont un hématocrite compris entre environ 70 et environ 90.

**3.** Procédé selon la revendication 1, dans lequel lesdits globules rouges d'hématocrite élevé ont un hématocrite d'environ 80.

**4.** Procédé selon la revendication 1, dans lequel lesdits globules rouges d'hématocrite élevé ont un hématocrite entre 80 et 95+.

**5.** Procédé selon la revendication 1, qui comprend en outre une étape de passage des globules rouges d'hématocrite élevé venant d'être séparés vers un conteneur de globules rouges intermédiaire avant ladite étape de passage des globules rouges d'hématocrite élevé à travers le filtre de leucoréduction.

**6.** Procédé selon la revendication 1, dans lequel ladite étape de passage des globules rouges d'hématocrite élevé venant d'être séparés à travers le filtre de leucoréduction a lieu sensiblement simultanément avec ladite étape de fourniture de globules rouges d'hématocrite élevé venant d'être séparés.

**7.** Procédé selon la revendication 1, dans lequel ladite étape de passage des globules rouges d'hématocrite élevé venant d'être séparés à travers le filtre de leucoréduction a lieu peu après ladite étape de fourniture de globules rouges d'hématocrite élevé venant d'être séparés.

**8.** Procédé selon la revendication 1, dans lequel ladite étape de passage a lieu dans un intervalle de zéro (0) à deux (2) heures de ladite étape de fourniture.

**9.** Procédé selon la revendication 1, dans lequel ladite étape de passage a lieu dans un intervalle de zéro (0) à quatre (4) heures de ladite étape de fourniture.

**10.** Procédé selon la revendication 1, dans lequel ladite étape de passage a lieu dans un intervalle de zéro (0) à huit (8) heures de ladite étape de fourniture.

**11.** Procédé selon la revendication 1, dans lequel ladite étape de passage a lieu dans un intervalle de zéro (0) à vingt-quatre (24) heures de ladite étape de fourniture.

**12.** Procédé selon la revendication 1, dans lequel ladite étape de fourniture de globules rouges d'hématocrite élevé venant d'être séparés comprend en outre :

la séparation des globules rouges d'autres composants sanguins en utilisant un système de séparation pour produire des globules rouges d'hématocrite élevé venant d'être séparés.

**13.** Procédé selon la revendication 12, dans lequel ledit système de séparation est un système d'aphérèse.

**14.** Procédé selon la revendication 12, dans lequel ledit système de séparation est un système de séparation centrifuge.

**15.** Procédé selon la revendication 12, dans lequel ledit système de séparation est un système basé sur une membrane.

**16.** Procédé selon la revendication 12, dans lequel ledit système de séparation est un système à écoulement sensiblement continu.

**17.** Procédé selon la revendication 12, dans lequel ledit système est un système en mode par lots.

**18.** Procédé selon la revendication 12, dans lequel ledit filtre de leucoréduction et ledit conteneur de collecte de globules rouges sont compris dans un ensemble de collecte de globules rouges.

**19.** Procédé selon la revendication 18, dans lequel ledit ensemble de collecte de globules rouges comprend en outre un réservoir de collecte de globules rouges intermédiaire qui est interposé entre ledit système de séparation et ledit filtre de leucoréduction ; le procédé comprenant en outre :

le passage des globules rouges d'hématocrite élevé séparés provenant dudit système de séparation dans le réservoir de globules rouges intermédiaire avant ladite étape de passage des globules rouges d'hématocrite élevé séparés à travers ledit filtre de leucoréduction.

**20.** Procédé selon la revendication 18, dans lequel ledit ensemble de collecte de globules rouges comprend en outre un ensemble de barrière/pointe stérile pour la connexion dudit ensemble de collecte de globules rouges à une source de solution de stockage ; ledit ensemble de barrière/pointe stérile étant interposé entre ledit système de séparation et ledit filtre de leucoréduction ; le procédé comprenant en outre
la connexion dudit ensemble de barrière/pointe stérile à une source de solution de stockage ; et
l'addition d'une solution de stockage aux globules rouges d'hématocrite élevé séparés après ladite étape de passage desdits globules rouges d'hématocrite élevé séparés à travers ledit filtre de leucoréduction.

**21.** Procédé selon la revendication 19, dans lequel ledit ensemble de collecte de globules rouges comprend en outre un ensemble de barrière/pointe stérile pour la connexion dudit ensemble de collecte de globules rouges à une source de solution de stockage ; ledit ensemble de barrière/pointe stérile étant connecté audit réservoir de globules rouges intermédiaire ; le procédé comprenant en outre
la connexion dudit ensemble de barrière/pointe stérile à une source de solution de stockage ; et
le passage d'une solution de stockage vers le réservoir de globules rouges intermédiaire et puis vers et à travers ledit filtre de leucoréduction et vers le conteneur de collecte pour se mélanger avec les globules rouges séparés après ladite étape de passage desdits globules rouges séparés.

**22.** Procédé selon la revendication 19, dans lequel ledit réservoir de globules rouges intermédiaire est connecté audit filtre de leucoréduction par un connecteur cassable ; moyennant quoi ledit connecteur cassable bloque l'écoulement de liquide à travers celui-ci ; le procédé comprenant en outre l'ouverture sélective dudit connecteur cassable pour permettre l'écoulement à travers celui-ci.

**23.** Procédé selon la revendication 18, dans lequel le conteneur de collecte de globules rouges est un premier réservoir de collecte de globules rouges et dans lequel ledit ensemble de collecte globules rouges comprend en outre un second réservoir de collecte de globules rouges qui est disposé après ledit filtre de leucoréduction ; le procédé comprenant en outre :

le passage des globules rouges d'hématocrite élevé séparés provenant dudit système de séparation dans et à travers ledit filtre de leucoréduction simultanément dans les premier et second réservoirs de collecte de globules rouges.

**24.** Procédé selon la revendication 18, dans lequel le conteneur de collecte de globules rouges est un premier réservoir de collecte de globules rouges et dans lequel ledit ensemble de collecte de globules rouges comprend en outre un second réservoir de collecte de globules rouges qui est disposé après ledit filtre de leucoréduction ; le procédé comprenant en outre :

le passage des globules rouges d'hématocrite élevé séparés provenant dudit système de séparation dans et à travers ledit filtre de leucoréduction, premièrement dans l'un parmi les premier et second réservoirs de collecte de globules rouges, et, deuxièmement, dans l'autre desdits premier et second réservoirs de collecte de globules rouges.

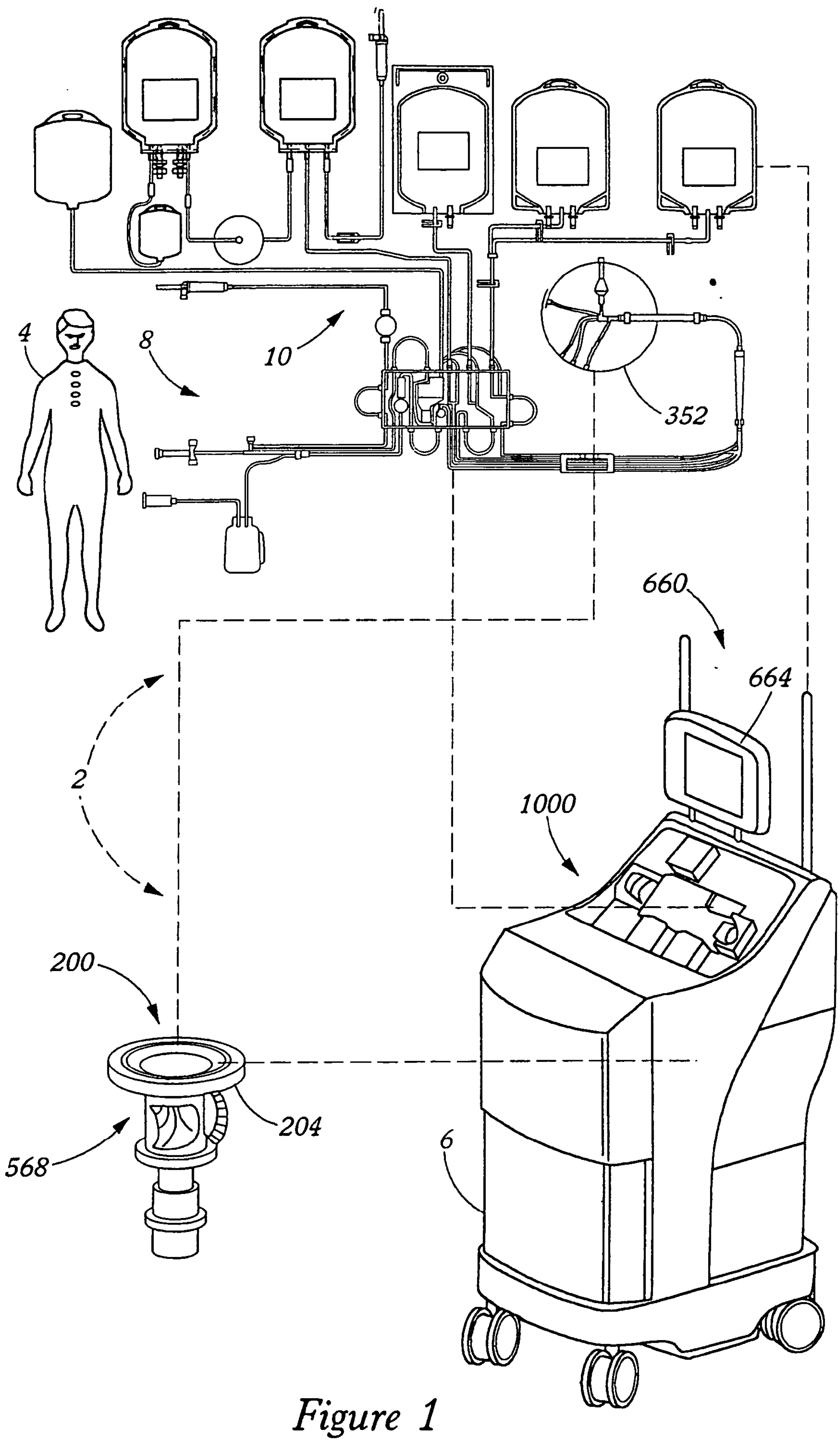


Figure 1

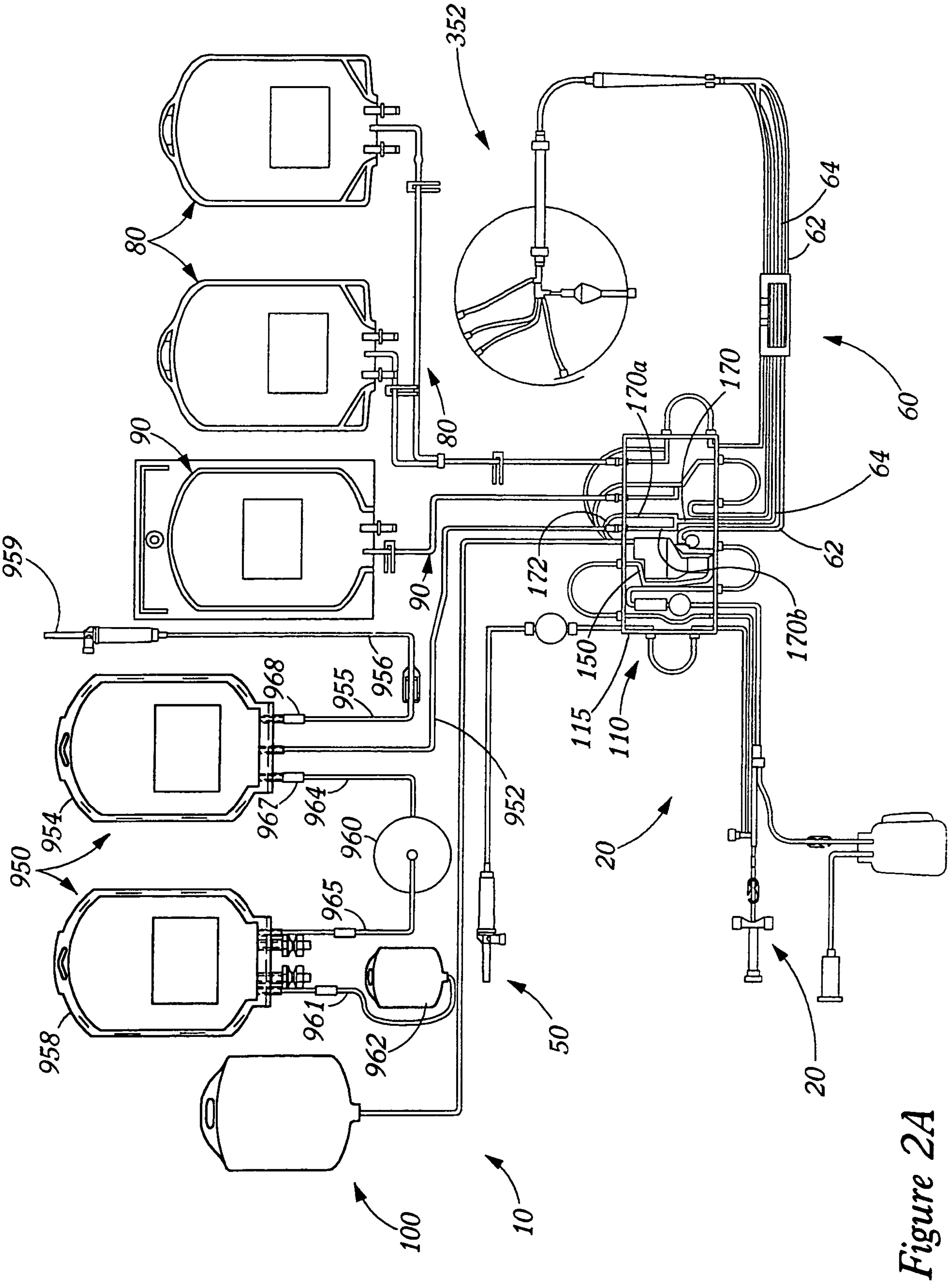
352
80
80
90
90
959
968
955
956
952
954
950
967
964
960
965
958
961
962
100
10
50
20
20
110
115
150
172
170a
170
170b
64
62
64
62
60

Figure 2A

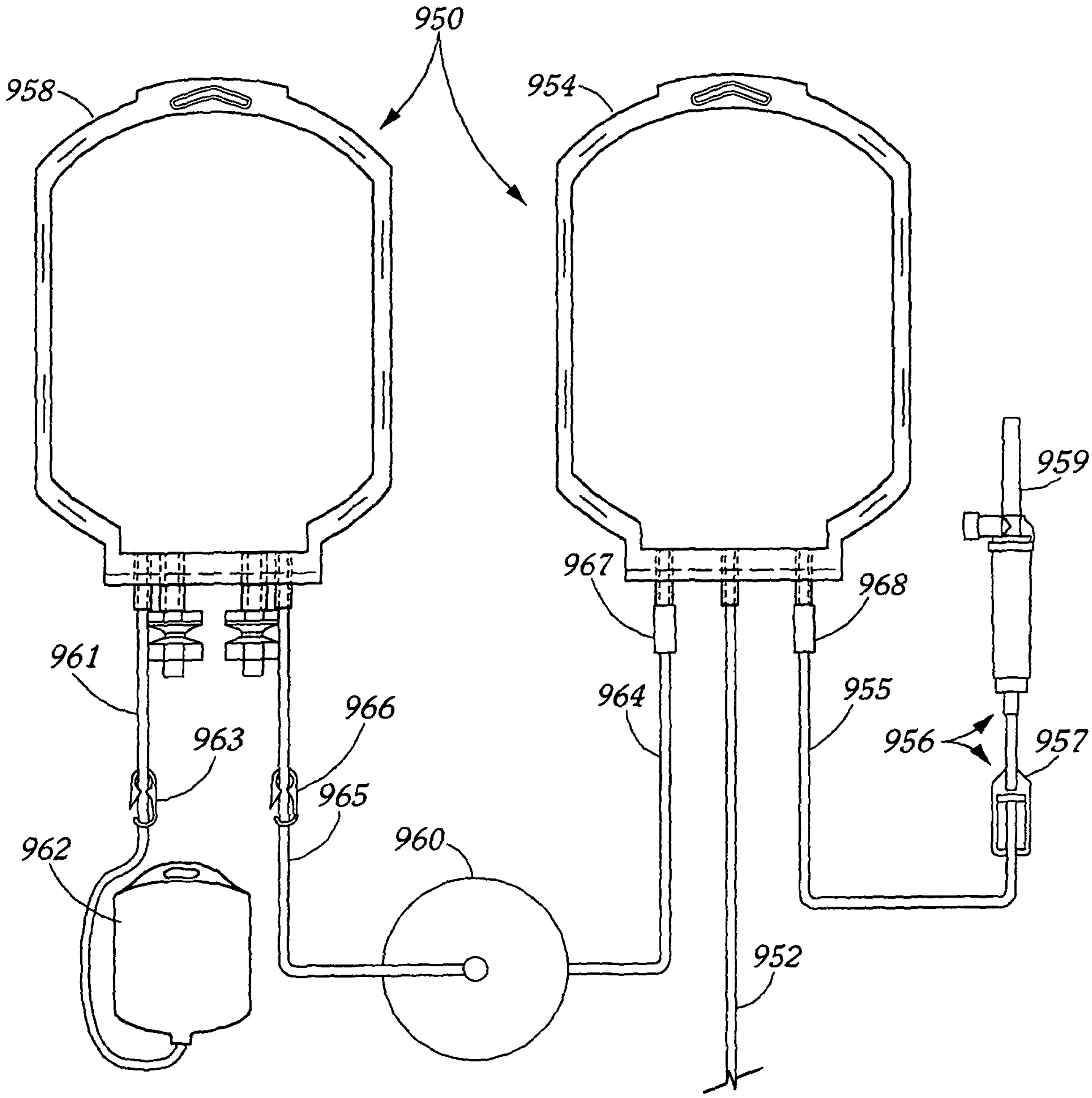
950
958
954
959
967
968
961
964
955
966
963
956
957
965
962
960
952

Figure 2B

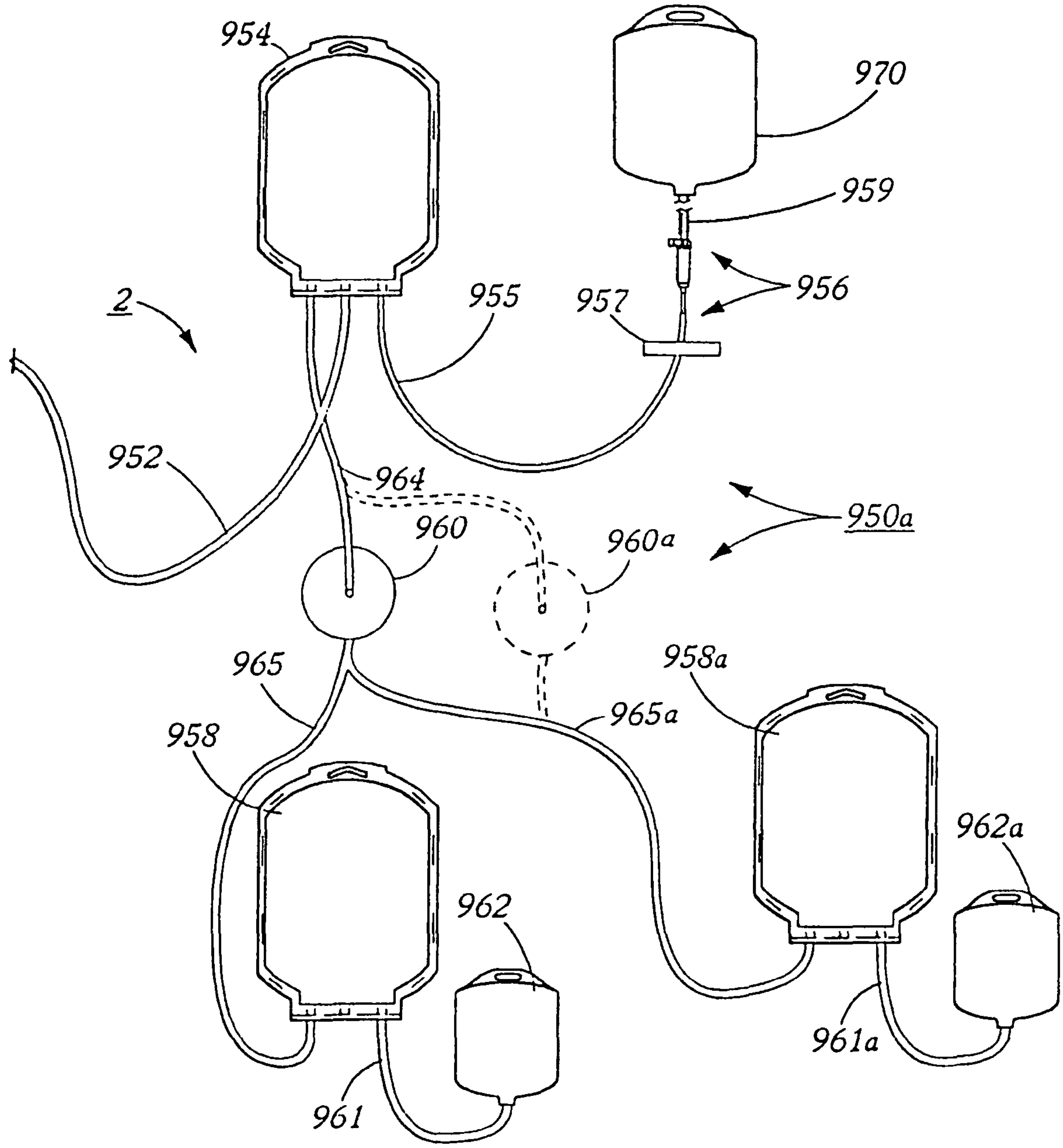
954
970
959
956
2
955
957
952
964
950a
960
960a
965
958a
965a
958
962a
962
961a
961

Figure 2C

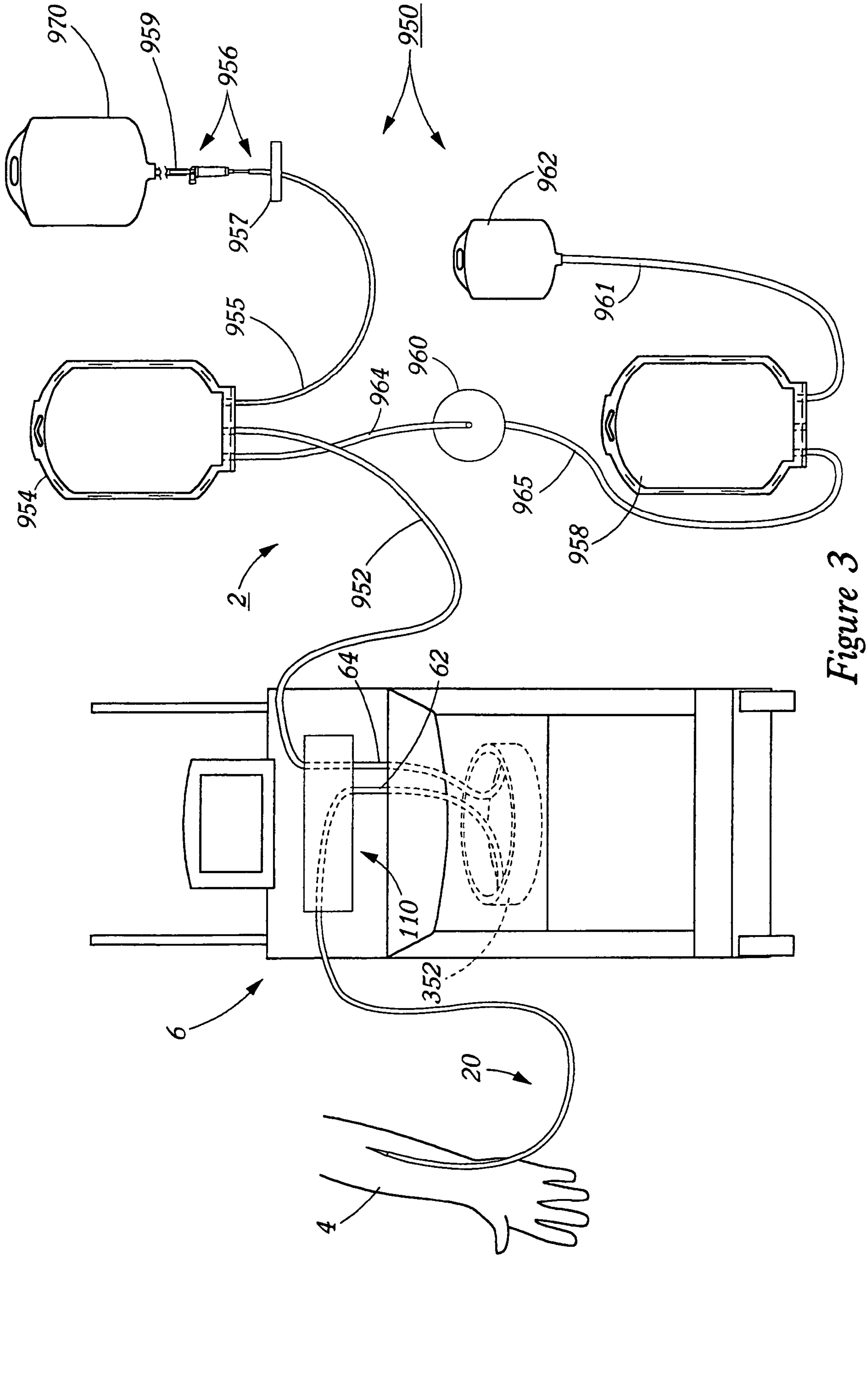
970
959
956
950
957
962
955
961
960
964
954
965
958
2
952
64
62
110
352
6
20
4

Figure 3

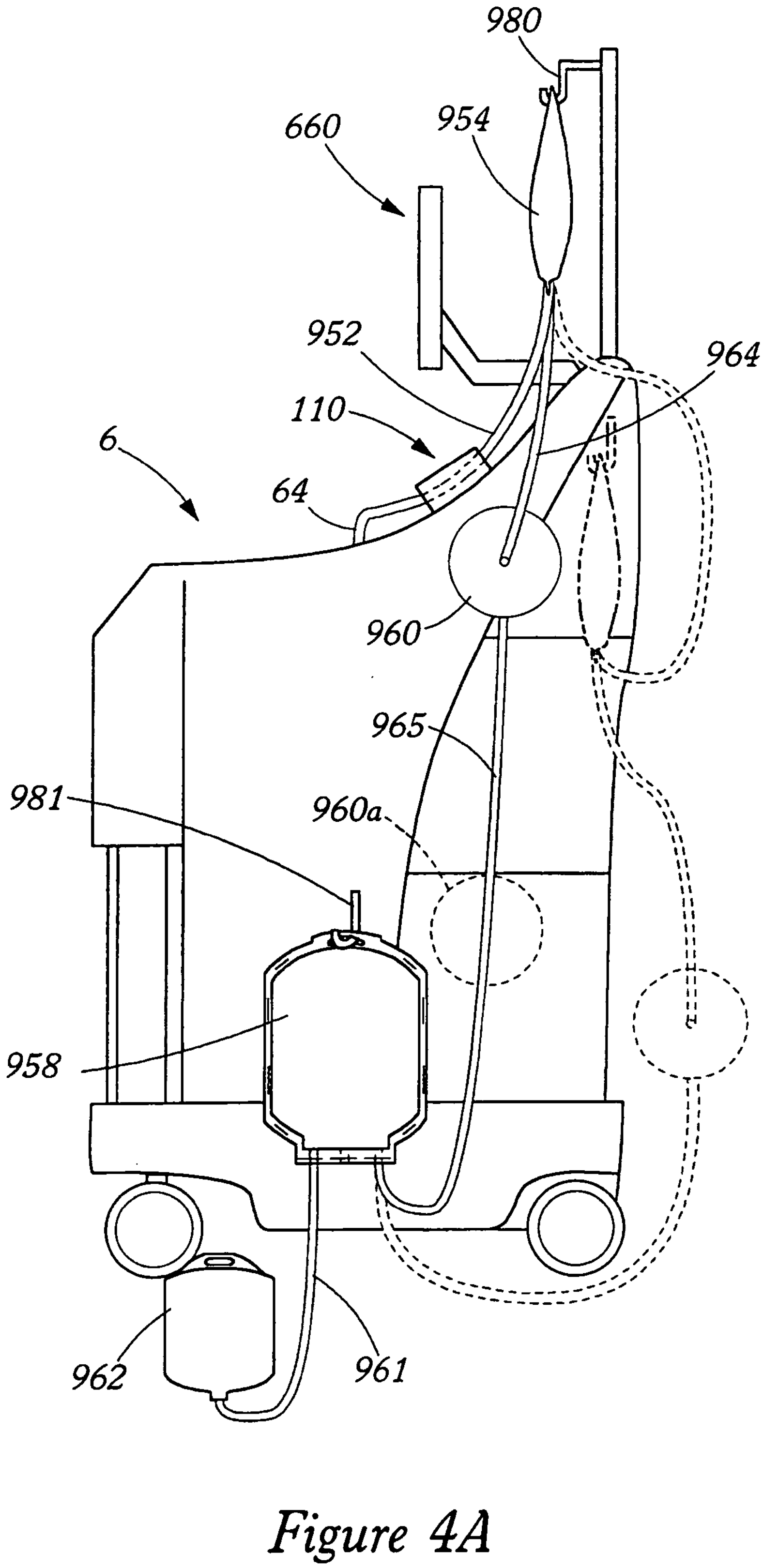


*Figure 4A*

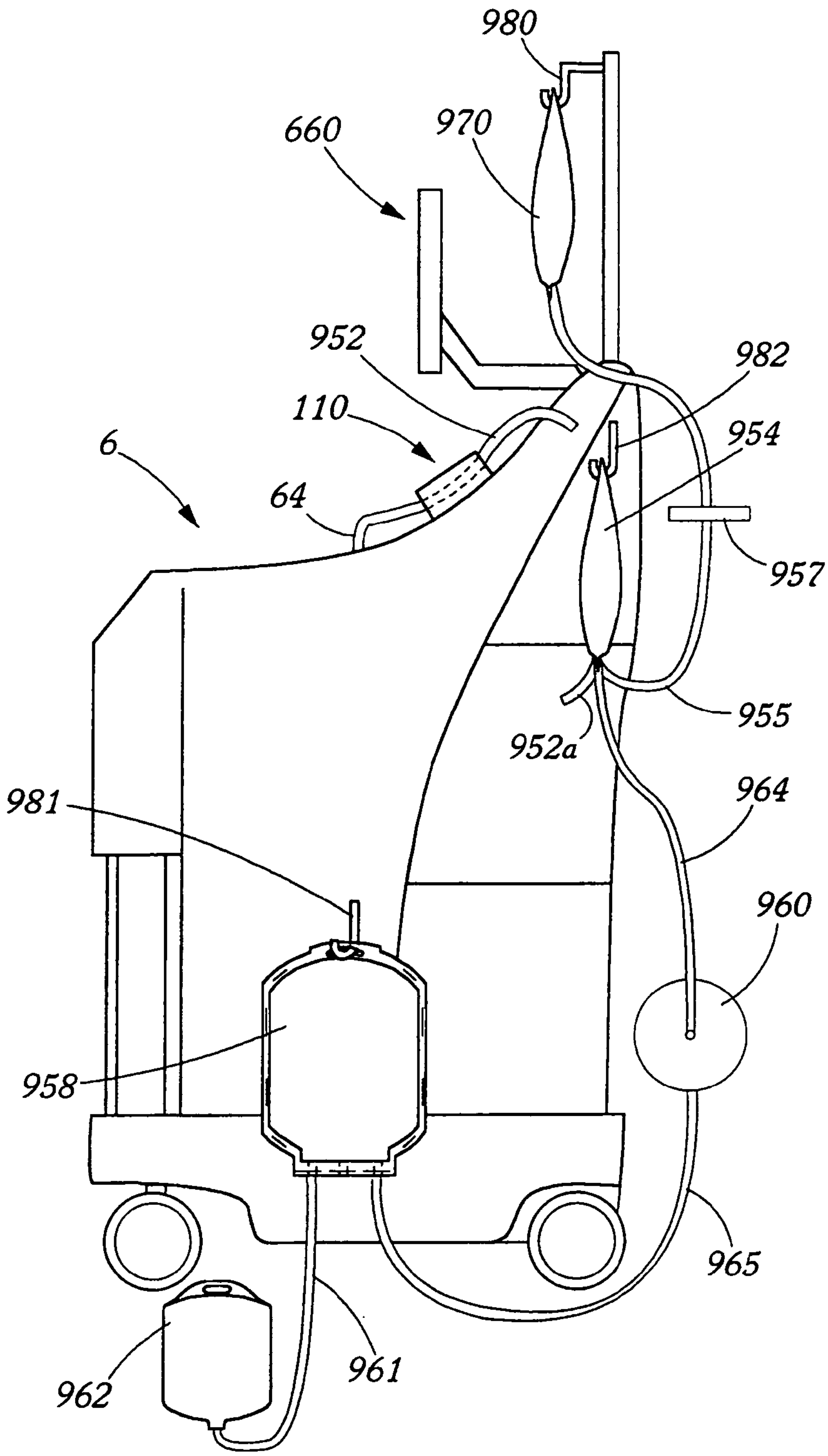
980
970
660
952
982
110
954
6
64
957
955
952a
964
981
960
958
965
961
962

Figure 4B

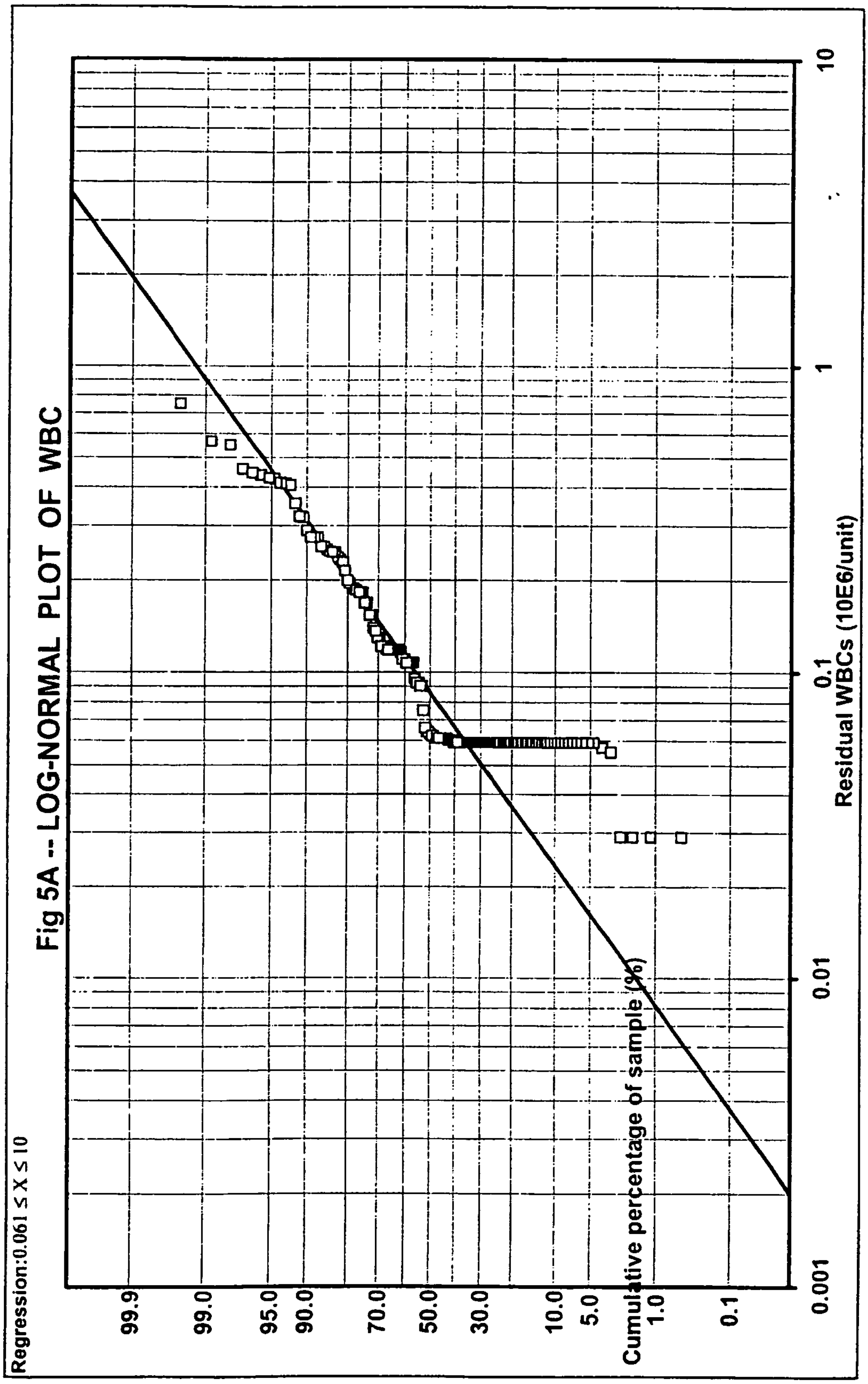
Fig 5A -- LOG-NORMAL PLOT OF WBC
Regression:0.061 ≤ X ≤ 10
Cumulative percentage of sample (%)
99.9
99.0
95.0
90.0
70.0
50.0
30.0
10.0
5.0
1.0
0.1
0.001
0.01
0.1
1
10
Residual WBCs (10E6/unit)

*Figure 5A*

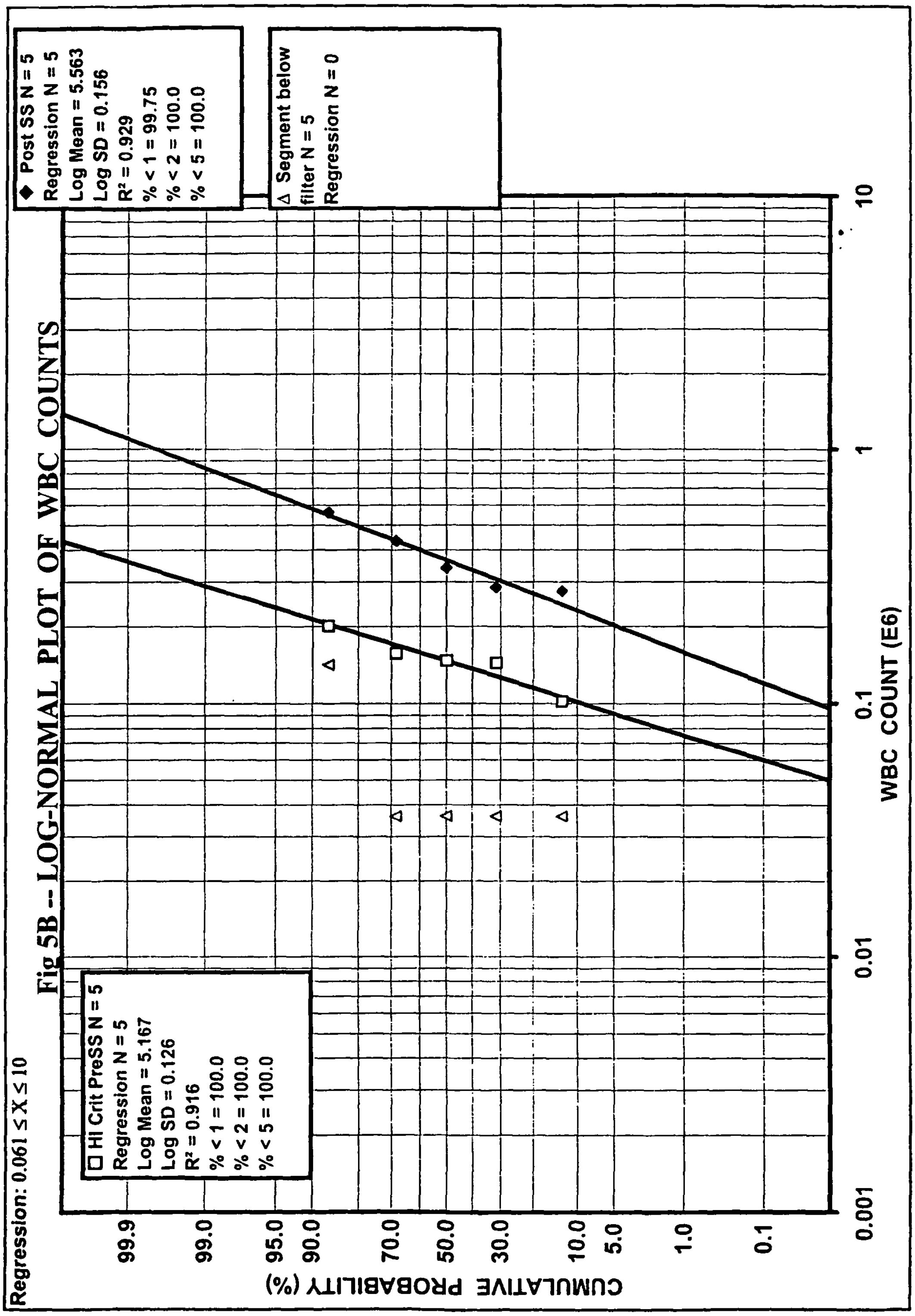
Regression: 0.061 ≤ X ≤ 10
Fig 5B -- LOG-NORMAL PLOT OF WBC COUNTS
◆ Post SS N = 5
Regression N = 5
Log Mean = 5.563
Log SD = 0.156
R² = 0.929
% < 1 = 99.75
% < 2 = 100.0
% < 5 = 100.0
Δ Segment below filter N = 5
Regression N = 0
□ HI Crit PreSS N = 5
Regression N = 5
Log Mean = 5.167
Log SD = 0.126
R² = 0.916
% < 1 = 100.0
% < 2 = 100.0
% < 5 = 100.0
CUMULATIVE PROBABILITY (%)
99.9
99.0
95.0
90.0
70.0
50.0
30.0
10.0
5.0
1.0
0.1
0.001
0.01
0.1
1
10
WBC COUNT (E6)

*Figure 5B*

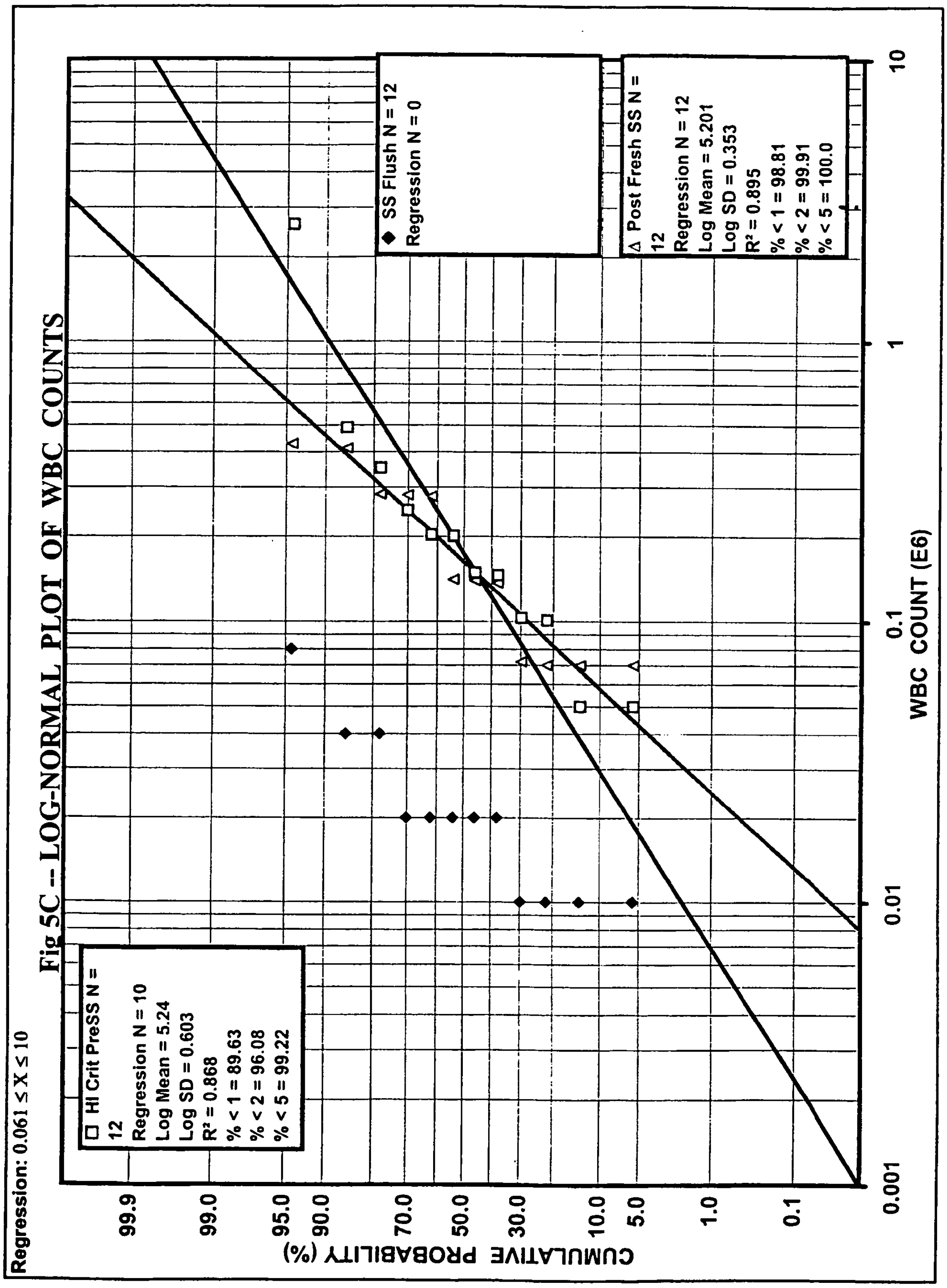
Regression: 0.061 ≤ X ≤ 10
Fig 5C -- LOG-NORMAL PLOT OF WBC COUNTS
□ HI Crit PreSS N = 12
Regression N = 10
Log Mean = 5.24
Log SD = 0.603
R² = 0.868
% < 1 = 89.63
% < 2 = 96.08
% < 5 = 99.22
◆ SS Flush N = 12
Regression N = 0
Δ Post Fresh SS N = 12
Regression N = 12
Log Mean = 5.201
Log SD = 0.353
R² = 0.895
% < 1 = 98.81
% < 2 = 99.91
% < 5 = 100.0
CUMULATIVE PROBABILITY (%)
99.9
99.0
95.0
90.0
70.0
50.0
30.0
10.0
5.0
1.0
0.1
0.001
0.01
0.1
1
10
WBC COUNT (E6)

*Figure 5C*

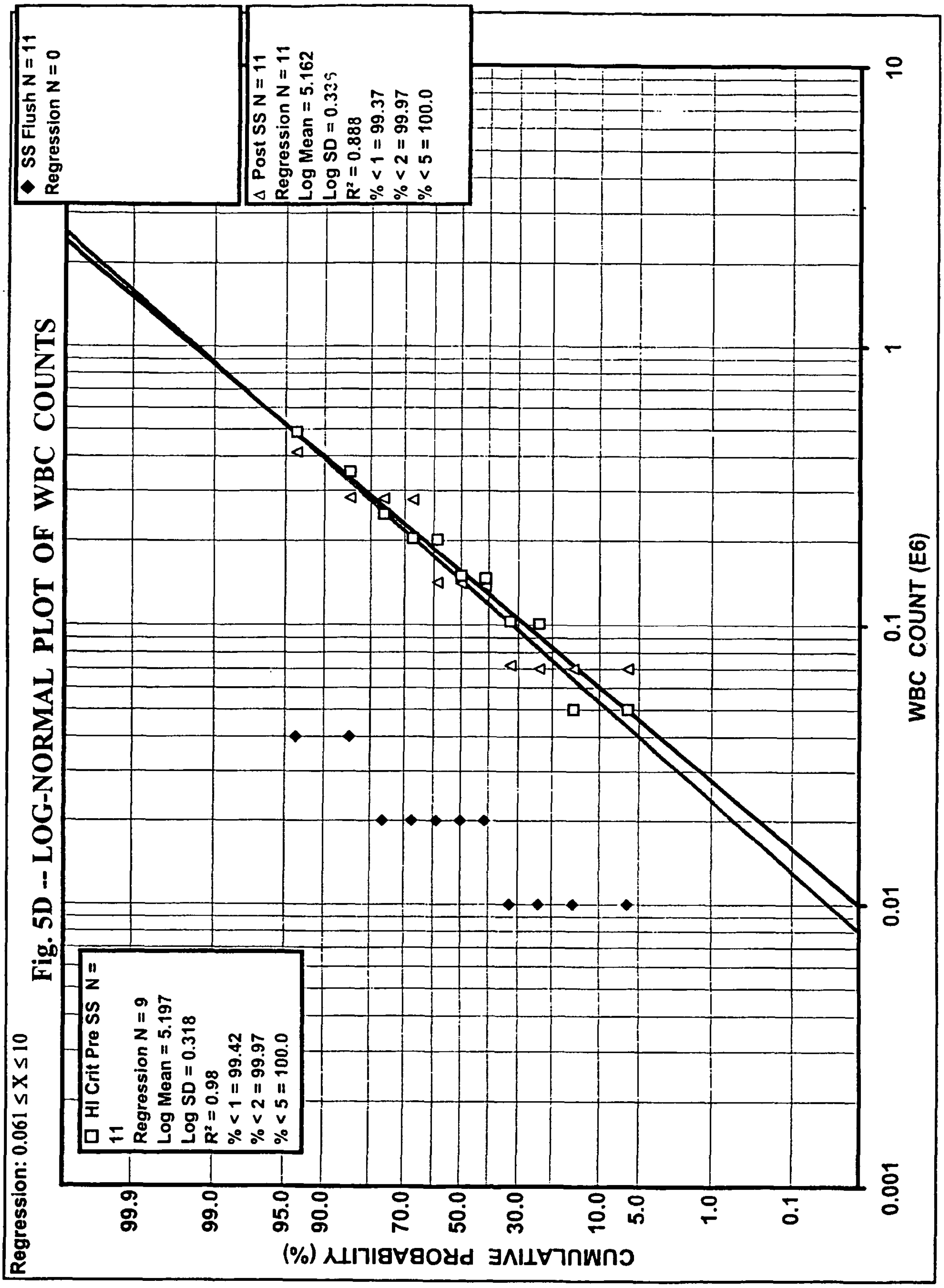
Regression: 0.061 ≤ X ≤ 10
Fig. 5D -- LOG-NORMAL PLOT OF WBC COUNTS
□ HI Crit Pre SS N = 11
Regression N = 9
Log Mean = 5.197
Log SD = 0.318
R² = 0.98
% < 1 = 99.42
% < 2 = 99.97
% < 5 = 100.0
◆ SS Flush N = 11
Regression N = 0
Δ Post SS N = 11
Regression N = 11
Log Mean = 5.162
Log SD = 0.339
R² = 0.888
% < 1 = 99.37
% < 2 = 99.97
% < 5 = 100.0
CUMULATIVE PROBABILITY (%)
99.9
99.0
95.0
90.0
70.0
50.0
30.0
10.0
5.0
1.0
0.1
0.001
0.01
0.1
1
10
WBC COUNT (E6)

*Figure 5D*

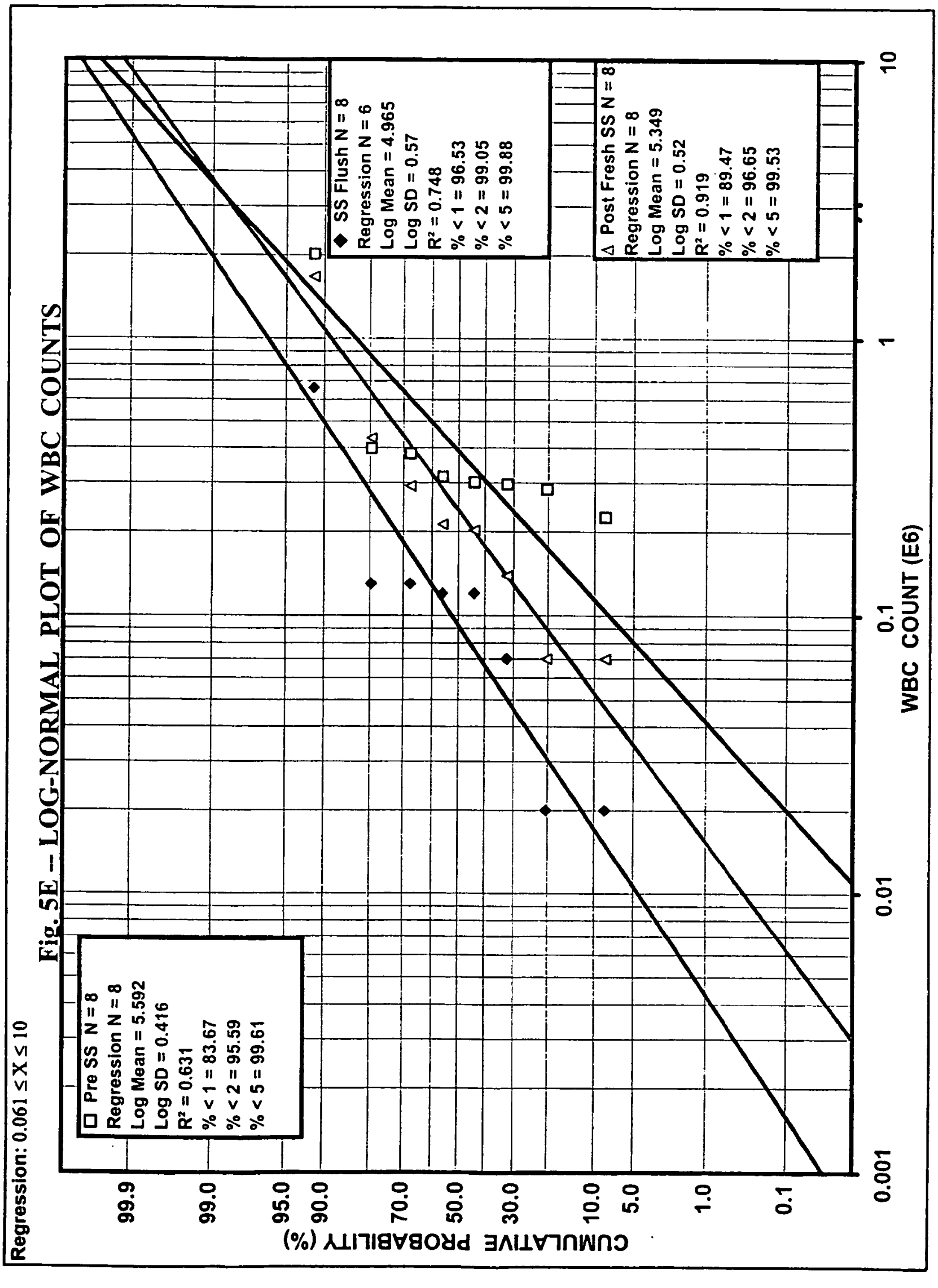
Regression: 0.061 ≤ X ≤ 10
Fig. 5E -- LOG-NORMAL PLOT OF WBC COUNTS
□ Pre SS N = 8
Regression N = 8
Log Mean = 5.592
Log SD = 0.416
R² = 0.631
% < 1 = 83.67
% < 2 = 95.59
% < 5 = 99.61
◆ SS Flush N = 8
Regression N = 6
Log Mean = 4.965
Log SD = 0.57
R² = 0.748
% < 1 = 96.53
% < 2 = 99.05
% < 5 = 99.88
Δ Post Fresh SS N = 8
Regression N = 8
Log Mean = 5.349
Log SD = 0.52
R² = 0.919
% < 1 = 89.47
% < 2 = 96.65
% < 5 = 99.53
CUMULATIVE PROBABILITY (%)
99.9
99.0
95.0
90.0
70.0
50.0
30.0
10.0
5.0
1.0
0.1
0.001
0.01
0.1
1
10
WBC COUNT (E6)

*Figure 5E*

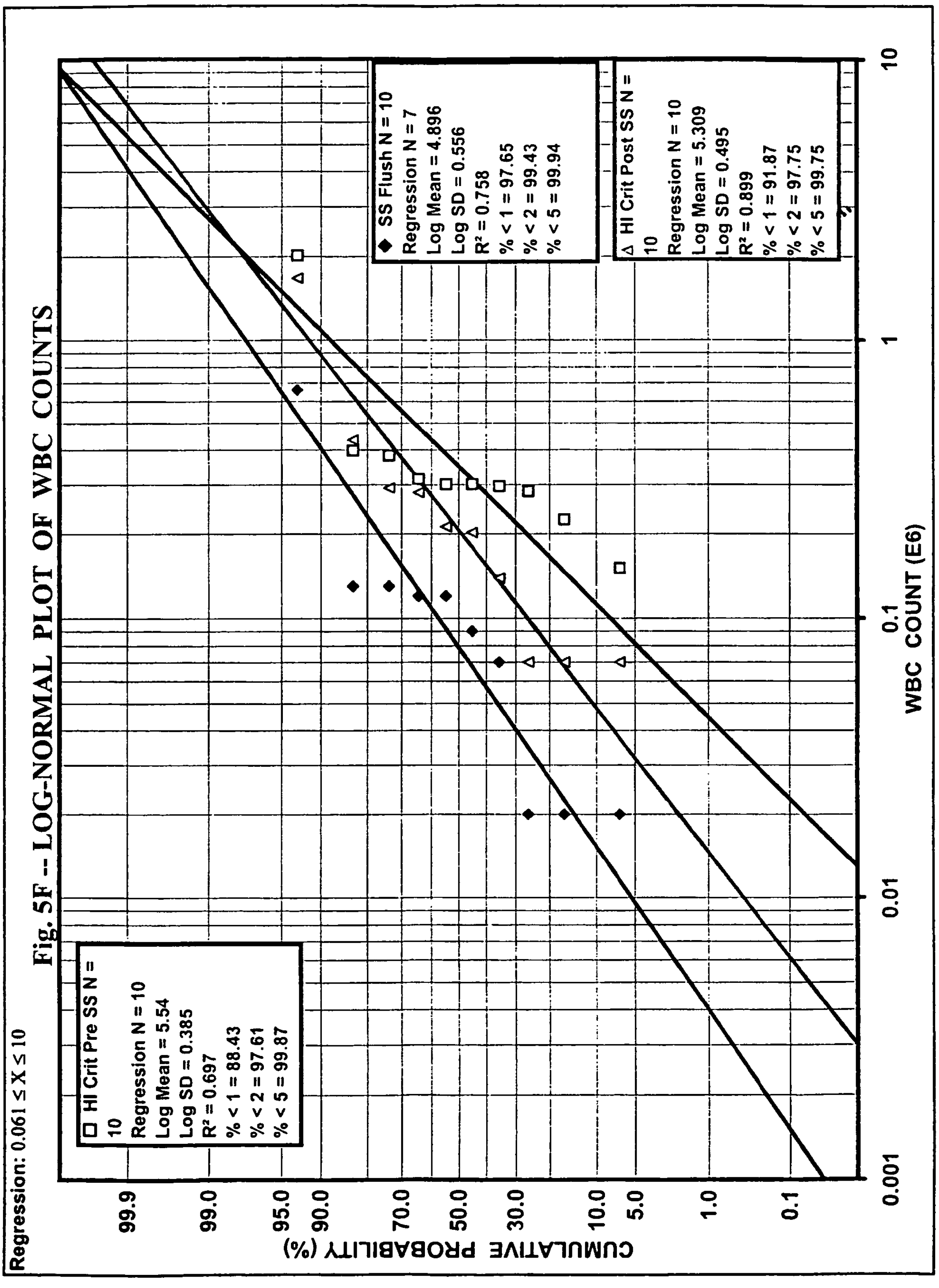
Regression: 0.061 ≤ X ≤ 10
Fig. 5F -- LOG-NORMAL PLOT OF WBC COUNTS
□ HI Crit Pre SS N = 10
Regression N = 10
Log Mean = 5.54
Log SD = 0.385
R² = 0.697
% < 1 = 88.43
% < 2 = 97.61
% < 5 = 99.87
◆ SS Flush N = 10
Regression N = 7
Log Mean = 4.896
Log SD = 0.556
R² = 0.758
% < 1 = 97.65
% < 2 = 99.43
% < 5 = 99.94
Δ HI Crit Post SS N = 10
Regression N = 10
Log Mean = 5.309
Log SD = 0.495
R² = 0.899
% < 1 = 91.87
% < 2 = 97.75
% < 5 = 99.75
99.9
99.0
95.0
90.0
70.0
50.0
30.0
10.0
5.0
1.0
0.1
CUMULATIVE PROBABILITY (%)
0.001
0.01
0.1
1
10
WBC COUNT (E6)

*Figure 5F*

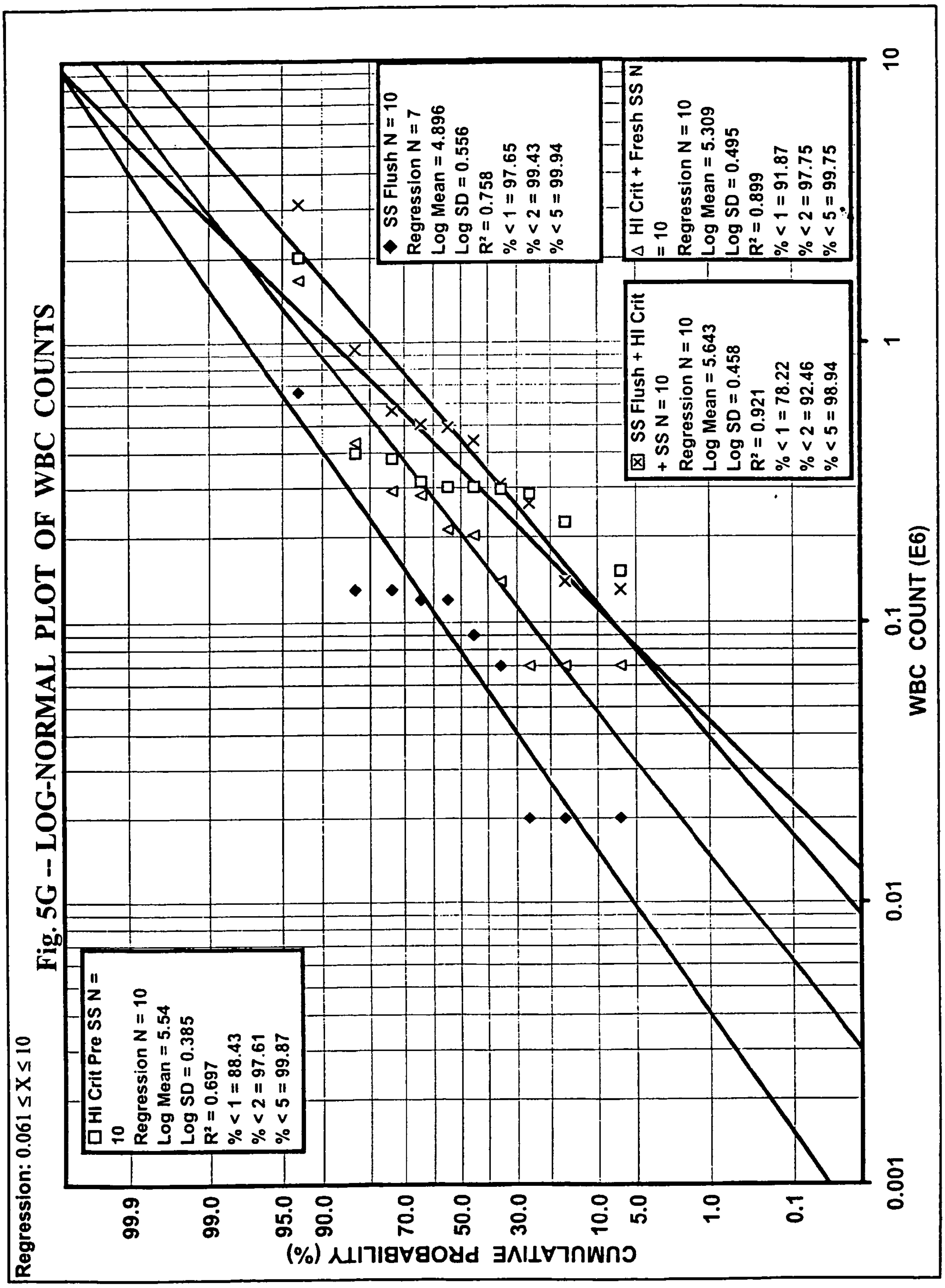
Regression: 0.061 ≤ X ≤ 10
Fig. 5G -- LOG-NORMAL PLOT OF WBC COUNTS
□ HI Crit Pre SS N = 10
Regression N = 10
Log Mean = 5.54
Log SD = 0.385
R² = 0.697
% < 1 = 88.43
% < 2 = 97.61
% < 5 = 99.87
◆ SS Flush N = 10
Regression N = 7
Log Mean = 4.896
Log SD = 0.556
R² = 0.758
% < 1 = 97.65
% < 2 = 99.43
% < 5 = 99.94
⊠ SS Flush + HI Crit + SS N = 10
Regression N = 10
Log Mean = 5.643
Log SD = 0.458
R² = 0.921
% < 1 = 78.22
% < 2 = 92.46
% < 5 = 98.94
Δ HI Crit + Fresh SS N = 10
Regression N = 10
Log Mean = 5.309
Log SD = 0.495
R² = 0.899
% < 1 = 91.87
% < 2 = 97.75
% < 5 = 99.75
CUMULATIVE PROBABILITY (%)
99.9
99.0
95.0
90.0
70.0
50.0
30.0
10.0
5.0
1.0
0.1
WBC COUNT (E6)
0.001
0.01
0.1
1
10

*Figure 5G*

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 5954971 A
- US 67251900 A
- WO 9911305 A
- WO 0124848 A
- US 5653887 A
- US 5676644 A
- US 5702357 A
- US 5720716 A
- US 5722946 A
- US 5738644 A
- US 5750025 A
- US 5795317 A
- US 5837150 A
- US 5919154 A
- US 5921950 A
- US 5941842 A
- US 6129656 A
- US 5345070 A
- US 5520218 A